# EUROPEAN PATENT SPECIFICATION

(11) **EP 3 307 914 B1**
(45) Date of publication and mention of the grant of the patent: **02.10.2019**
(21) Application number: 16731956.5
(22) Date of filing: 08.06.2016
(51) Int. Cl.: C12R 1/01, A01N 63/00, C12N 15/113, C12N 15/82

(54) **PEST CONTROL SYSTEM**
SCHÄDLINGSBEKÄMPFUNGSSYSTEM
SYSTÈME DE LUTTE CONTRE LES INSECTES NUISIBLES

(30) Priority: 11.06.2015 GB 201510154
(43) Date of publication of application: 18.04.2018
(73) Proprietor: Swansea University, Swansea SA2 8PP (GB)
(72) Inventor: DYSON, Paul, Swansea West Glamorgan SA2 8PP (GB); WHITTEN, Miranda, Swansea SA2 8PP (GB)
(74) Representative: Myint, Julie Marie
(86) International application number: PCT/GB2016/051683
(87) International publication number: WO 2016/198852

(56) References cited:
- WO-A1-2013/117910
- US-A1- 2006 021 087
- MABEL L. TARACENA ET AL: "Genetically Modifying the Insect Gut Microbiota to Control Chagas Disease Vectors through Systemic RNAi", PLOS NEGLECTED TROPICAL DISEASES, vol. 9, no. 2, 12 February 2015 (2015-02-12), page e0003358, XP055292472, US ISSN: 1935-2727, DOI: 10.1371/journal.pntd.0003358
- MIRANDA M. A. WHITTEN ET AL: "Insect gut nucleases: a challenge for RNA interference mediated insect control strategies", PROCEEDINGS - ROYAL SOCIETY, BIOLOGICAL SCIENCES, vol. 1, no. 1825, 24 February 2016 (2016-02-24), page 198, XP055292452, GB ISSN: 0962-8452, DOI: 10.1016/j.jinsphys.2013.08.014
- KATHERINE A. MURPHY ET AL: "Ingestion of genetically modified yeast symbiont reduces fitness of an insect pest via RNA interference", SCIENTIFIC REPORTS, vol. 6, 2 March 2016 (2016-03-02), page 22587, XP055292453, DOI: 10.1038/srep22587
- BADILLO-VARGAS ISMAEL E ET AL: "RNA interference tools for the western flower thrips,Frankliniella occidentalis", JOURNAL OF INSECT PHYSIOLOGY, vol. 76, 18 March 2015 (2015-03-18), pages 36-46, XP029226698, ISSN: 0022-1910, DOI: 10.1016/J.JINSPHYS.2015.03.009
- M A SHAH ET AL: "RNA INTERFERENCE FOR INSECT PESTMANAGEMENT-RECENT DEVELOPMENTS: A REVIEW", JOURNAL CELL TISSUE RESEARCH, 1 October 2014 (2014-10-01), pages 4601-4608, XP055292686,
- SCOTT JEFFREY G ET AL: "Towards the elements of successful insect RNAi", JOURNAL OF INSECT PHYSIOLOGY, vol. 59, no. 12, 13 September 2013 (2013-09-13), pages 1212-1221, XP028778830, ISSN: 0022-1910, DOI: 10.1016/J.JINSPHYS.2013.08.014

## Description

### Field of the Invention

The invention relates to a genetically transformed or transfected bacterial cell that is a gut symbiont of an insect belonging to the Order *Thysanoptera* wherein said cell is transformed to express double-stranded RNA (dsRNA) active against at least one selected insect gene; a vector for transforming or transfecting said bacterial cell; an insect including said transformed bacterial cell and a method of pest control employing the use of said bacterial cell and/or said insect.

### Background of the Invention

Invertebrates and other pests are common vectors for pathogenic organisms, typically micro-organisms that are responsible for a variety of diseases that can affect crops.

Over the past 30 years, insects such as western flower thrips (WFT), *Frankliniella occidentalis* (Pergande) (Thysanoptera: Thripidae), has become one of the most important agricultural pests worldwide. Its pest status can be attributed to several factors, including its reproductive potential, invasiveness (endemic to Western North America, it has invaded many countries in Europe, Africa and Asia), range of host crops, ability to transmit plant viruses, and insecticide resistance. All of these factors are interrelated, and are linked to the life cycle and life history strategy of the species. It is a significant pest of greenhouse and field crops and soft fruit in Europe, and staples such as beans, cowpeas and groundnuts in Africa and Asia and this can have devastating effects on humans and animals that rely on those crops for nutrition.

Direct crop damage results from both feeding and damage of plants by way of oviposition. Thrips also transmit several different tospoviruses, including Groundnut ringspot virus (GRSV) and Tomato spotted wilt virus (TSWV) and it has been estimated that TSWV alone causes over $1 billion in losses annually. Over 1,000 species of plants in 84 families are susceptible to TSWV, giving it one of the broadest host ranges of any plant pathogen.

Consequently, there is a continued interest in developing control methods aimed at reducing or eradicating the incidence of the above agricultural pests. This is often achieved either by targeting the pathogen itself (GRSV/TSWV) or the vector (WFT) commonly associated with transmission. Methods for controlling infestations and infection by insects have typically been in the form of; physical barriers preventing transmission; chemical compositions, such as insecticides, chemical drugs and repellents; and also biological controls.

Recent advances in genetics has led to a greater understanding of the development of a vast range of organisms, and paved the way for new avenues in biological pest control. The study of insect gene function provides a crucial step towards understanding the physiology, behaviour, immunology and even disease transmission in this very diverse and successful group of organisms. Armed with this knowledge it is possible to develop ways to fight crop damage and develop strategies to control pest insect populations.

RNA interference (RNAi) is a powerful technique of sequence-specific down-regulation of gene expression to interrogate eukaryotic gene function on an individual gene basis.

RNAi is a form of post-transcriptional gene silencing wherein a specific mRNA of a particular gene is destroyed or blocked, preventing translation and formation of an active gene product. RNAi occurs naturally within living cells to modulate gene activity, and is also important in defence against parasites and viral infection. For example, when a cell is injected with RNA in a double-stranded (ds) form, a protein called Dicer (or RNase III) cleaves the dsRNA molecules into short fragments of RNA (20-25 nucleotides), termed short interfering RNA (siRNA) due to their ability to interfere with the expression of a specific gene. These siRNA molecules are unwound into single stranded (ss) RNA, whereupon the so-called guide strand is incorporated into the RNA-induced silencing complex (RISC). Often, this guide strand will base pair with a complimentary sequence of mRNA in the cell inducing its cleavage by the catalytic component of the RISC complex. The mRNA is not translated and no functional protein is produced, and therefore the effects of the gene encoding the specific mRNA are 'silenced'. This process is termed cell-autonomous RNAi, wherein gene silencing is limited to the cell in which the dsRNA is introduced. Alternatively, environmental and systemic RNAi are the two forms of non-cell autonomous RNAi, wherein the interfering effect takes place in cells/tissues different from where the dsRNA was introduced/produced. In this case, the dsRNA is either taken up into multiple cells (environmental RNAi such as in viral infections), or the silencing signal is transported from the cell in which the dsRNA is applied or expressed to other cells where the effect is observed (systemic RNAi).

By artificially synthesising dsRNA (or siRNA molecules) with a known sequence complimentary to a gene of interest, and introducing it to target cells, it is possible to understand the role of a specific gene by observing the consequences of its loss of activity. RNAi and other so-called reverse genetics techniques are thus revolutionizing biological sciences, with applications in genomics, biotechnology, and medicine.

RNAi in invertebrates is an established technology, wherein dsRNA is delivered most commonly by injection. However, this process often has high mortality rates due to injection trauma and anaesthesia, and also requires high sample numbers. Large insects also require expensive quantities of dsRNA to be synthesised. Moreover, as stated, this results in cell-autonomous gene silencing achieving transient RNAi effects and, therefore, is not applicable for the control of insect pests in the field. As a result, for efficient insect pest control non-cell autonomous RNAi is required, which has been shown to be achieved by feeding insects with a biological source such as genetically modified bacteria or plant material. This therefore begins with the uptake of dsRNA environmentally into the gut lumen of the insect, from where it spreads to tissues elsewhere (systemic RNAi). Achieving RNAi depends on a reliable method for delivering, or uptake of, a dsRNA copy of part of a target gene to the insect. For some insect species this has been achieved by including in their food live or dead *E*. *coli* cells expressing dsRNA. The bacteria are digested in the gut and the dsRNA is taken up and delivered systemically to different tissues where it can mediate transient RNAi. EP2374462A2 teaches that RNAi can be introduced by ingestion of: naked dsRNA, food contaminated with *E*. *coli* expressing dsRNA e.g. by spraying with transformed bacteria, or genetically modified plant material expressing dsRNA. WO2011017137A2 teaches a method whereby a food bait of the insect is contaminated with genetically modified bacteria expressing dsRNA and the bait is returned to a colony to be fed on by the insects. Further, WO2011025860A1 teaches the use of RNAi against plant-feeding insects, wherein bacteria that infect specific plants are genetically modified to express specific dsRNA. Similarly, WO2011036536A2 teaches specific RNAi gene targets which are silenced by the delivery of dsRNA by spraying dead bacteria onto crop plants.

However, many of the currently developed techniques are often impractical or have poor efficacy and are not targeted at organisms that cause particular harm to the environment. For example, contamination of food sources (plant or other) with genetically modified bacteria may be harmful toward other insects that may be occasional feeders. Furthermore, spraying chemical compositions containing naked dsRNA onto insects or their food source may also have implications on other non-pest organisms. More importantly, all of the existing methods only teach delivery methods whereby transient gene-silencing effects are observed. Many of the currently employed techniques only exhibit short silencing durations, which are often too transient for certain targets e.g. those for hormone and developmental studies. Therefore re-application of dsRNA (or other RNAi constructs) is often required, which is costly and time-consuming. Furthermore, as currently employed techniques are not transferrable, effective pest control has been difficult to demonstrate. Therefore the technology in its current state is inappropriate for many insect species and improved efficacy and methods of application are required.

We have therefore developed a new RNAi technique effective against insects belonging to the Order *Thysanoptera* that relies on the *in vivo* synthesis of dsRNA by transgenic symbiotic gut bacteria from a species that naturally reside in the insect. Moreover, we developed a new RNAi technique that kills the insects in both the larval and adult stage but particularly the larval stage thus, advantageously, before insect pest reproduction and feeding occurs and before transmission of plant pathogens between individual plants occurs.

### Statements of Invention

According to a first aspect of the invention there is therefore provided a genetically transformed or transfected bacterial cell wherein said bacteria is a gut symbiont of an insect belonging to the Order *Thysanoptera* characterised in that said bacterial cell is transformed to express dsRNA against at least a part of tubulin gene or at least a part of elongation factor gene of the insect.

In a preferred embodiment of the invention said bacterial cell is transformed to express dsRNA against at least a part of tubulin alpha-1 chain gene or at least a part of elongation factor 1-alpha gene of the insect.

Reference herein to tubulin alpha-1 chain gene is to the gene having accession number GT305545 (GenBank; NCBI). and reference herein to elongation factor 1-alpha gene is to the gene having accession number GT303726 (GenBank; NCBI).

In a preferred embodiment of the invention, said dsRNA comprises a strand of RNA that shares 50% complementarity to at least a part of said tubulin gene or at least a part of elongation factor gene. It is preferred that said dsRNA comprises a strand of RNA that shares at least 75% complementarity to at least one of said genes of said insect and, in increasing order of preference, at least 80%, 85%, 90%, 91%, 92%, 93%, 94%, 95%, 96%, 97%, 98% or 99% complementarity to at least one of said target genes of said insect.

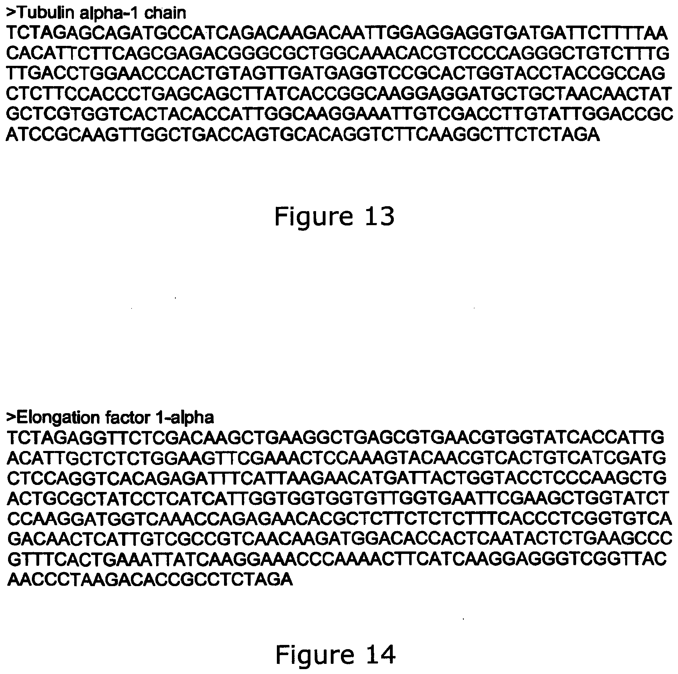

More ideally still said dsRNA active against said tubulin alpha-1 chain gene or said elongation factor 1-alpha gene of the insect is complementary to at least a part of the sequence structure shown in Figure 13 or 14, respectively. It is preferred that said dsRNA comprises a strand of RNA that shares at least 75% complementarity to at least a part of the sequence structure shown in Figure 13 or 14 and, in increasing order of preference, at least 80%, 85%, 90%, 91%, 92%, 93%, 94%, 95%, 96%, 97%, 98% or 99% complementarity to at least a part of the sequence structure shown in Figure 13 or 14.

Most ideally said bacteria is related to the genus *Pantoea,* which is a gut symbiont of said insect, ideally, said bacteria is BFo2, with a genome sequence having accession number SAGS00000000 (GenBank, NCBI). Alternatively, said said bacteria belongs to the genus *Erwinia,* which is a gut symbiont of said insect, ideally, said bacteria is BFo1, with a genome sequence having accession number LAGP00000000 (GenBank, NCBI).

More preferably, said insect is a member of the *Thripidae family.* More preferably still, said insect belongs to the genus *Frankliniella* such as the species *Frankliniella occidentalis.*

In yet a further preferred embodiment of the invention there is provided an expression vector for transforming or transfecting said bacterial cell wherein said vector comprises a nucleic acid sequence that expresses dsRNA against at least a part of said tubulin gene or at least a part of said elongation factor gene of the insect.

In a preferred embodiment of the invention said expression vector expresses dsRNA against at least a part of tubulin alpha-1 chain gene or at least a part of elongation factor 1-alpha gene of the insect.

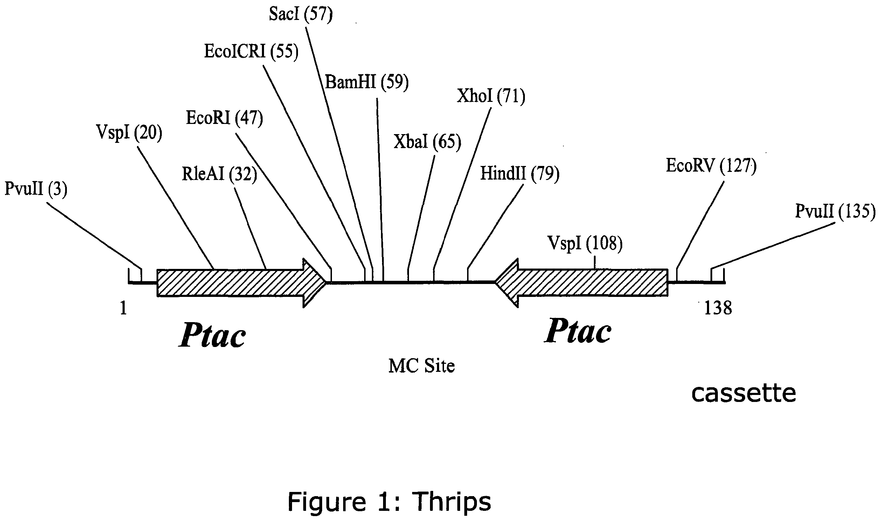

More preferably still said expression vector for transforming or transfecting said bacterial cell comprises at least one constitutive promoter, for example *Ptac.* Yet more preferably a plurality, such as a pair, of said promoters are provided and configured to drive transcription in a convergent manner to ensure transcription from both complementary strands of nucleic acid that expresses said dsRNA. Yet more preferably said expression vector for transforming or transfecting said bacterial cell is configured as shown in Figure 1 i.e. configured as per the Pex-A vector. More ideally still said expression vector for transforming or transfecting said bacterial cell is pTub3 or pElong1. Advantageously, pTub3 or pElong1 comprise nucleic acid sequences that are templates for dsRNA synthesis which are flanked by convergent *Ptac* promoters to ensure constitutive transcription of two complementary RNA strands that hybridise to generate the desired double stranded RNA.

In a preferred embodiment of the invention, said bacterial cell is transformed or genetically modified such that recombinant DNA is stably integrated into the host cell genome. This advantageously ensures long-term target gene silencing and ensures spread in insect populations. Ideally, stable integration is achieved by way of site specific integration, typically following the use of conventional site specific integration sites. Preferably, site specific integration is achieved in the *RNaseIII* gene.

Those skilled in the art will be aware said insect causes a disease in plants typically by the transmission of pathogenic organism belongs to the *tospoviruses* including Groundnut ringspot virus or tomato spotted wilt virus.

The working of the invention is particularly effective because the insect engages in horizontal gut transfer i.e. the acquisition of gut flora bacteria by ingestion from an environmental source. Thus, working of the invention is particularly effective because said horizontal gut transfer is achieved by ingestion of faeces or frass from other insects. More ideally still, ingestion of faeces or frass is genus-specific, and believed to be species-specific, whereby the parent generation of a species transfers the genetically engineered gut symbiont to an off spring generation. In this way, said insect can acquire said transformed or transfected bacterial cell from contaminated faeces or frass in the environment, circumventing the need for insect handling and associated mortality. Furthermore, advantageously, this permits horizontal transfer of dsRNA mediating RNAi throughout at least one insect colony and, typically, many insect colonies.

Additionally, or alternatively to the site specific integration mentioned above, in yet a further preferred embodiment of the invention, said transformed or transfected bacterial cell is also genetically engineered such that it does not produce functional RNA degrading proteins, including but not limited to, RNase III. Advantageously, this minimises the risk of enzymatic degradation of said dsRNA encoded by the transformed bacterial cell. Ideally, this is by deleting the whole or a part of native RNase III gene and, ideally replacing it with an antibiotic resistant gene, for example, an apramycin resistance gene.

Advantageously, targeting of the tubulin alpha-1 chain gene or elongation factor 1-alpha gene results in early mortality of said insect. In this way, the insect's capacity to both transmit a pathogenic micro-organism is reduced and damage to foliage due to feeding or egg laying is limited.

In use, the insect acquires the dsRNA genetically transformed or transfected gut symbiont bacterial cell from its environment through ingestion. Said bacterial cell thereby establishes itself as a living population in the gut of the insect, wherein it divides and actively transcribes the dsRNA which it encodes. Advantageously, this therefore mediates RNAi in the insect indefinitely. The dsRNA is ideally targeted against tubulin alpha-1 chain gene or elongation factor 1-alpha leading to its modulation and more specifically, its down regulation. Given the importance of these genes the insect is suitably compromised and so killed.

According to a second aspect of the invention there is provided an insect belonging to the Order *Thysanoptera* characterised in that said insect comprises a genetically transformed or transfected bacterial cell wherein said bacteria is a gut symbiont of said insect and is transformed to express dsRNA against at least a part of tubulin alpha-1 chain gene or at least a part of elongation factor 1-alpha gene of the insect.

Ina preferred embodiment of the invention said bacteria is transformed or transfected to express dsRNA against at least a part of tubulin alpha-1 chain gene or at least a part of elongation factor 1-alpha gene of the insect.

According to a third aspect of the invention, there is provided the use of the afore described bacterial cell in a method for modulating expression of a target gene of an insect belonging to the Order *Thysanoptera* comprising:
contaminating a composition to be ingested by the insect with said bacterial cell;
whereupon ingestion of said bacterial cell by said insect results in said bacterial cell colonising the gut of said insect wherein it synthesises dsRNA against at least a part of tubulin gene or at least a part of elongation factor gene to modulate said insect gene expression.

In a preferred use of said invention said bacterial cell is transformed or transfected to express dsRNA against at least a part of tubulin alpha-1 chain gene or at least a part of elongation factor 1-alpha gene of the insect.

In a preferred embodiment of the invention, said composition comprises a food source for the insect. More preferably still, said composition is the faeces or frass of said insect, or a food source of the juvenile insect such as, but not limited to, a plant source.

In a further preferred embodiment of the third aspect of the invention, modulating said target gene expression kills said insect, ideally at the larval stage, and so is an effective method of pest control or elimination.

In the claims which follow and in the preceding description of the invention, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.
The invention will now be described by way of example only with reference to the following figures wherein :-
**Figure 1****: shows the configuration of a Thrips cassette.** Converging promoters are indicated in red. Notice that *Ptac* promoters do not contain an operator sequence, therefore are not subject to Lacl repression.
**Figure 2****: shows a Pex-A vector map** (Eurofins, http://www.operon.com/products/gene-synthesis/ListOfVectors.aspx).

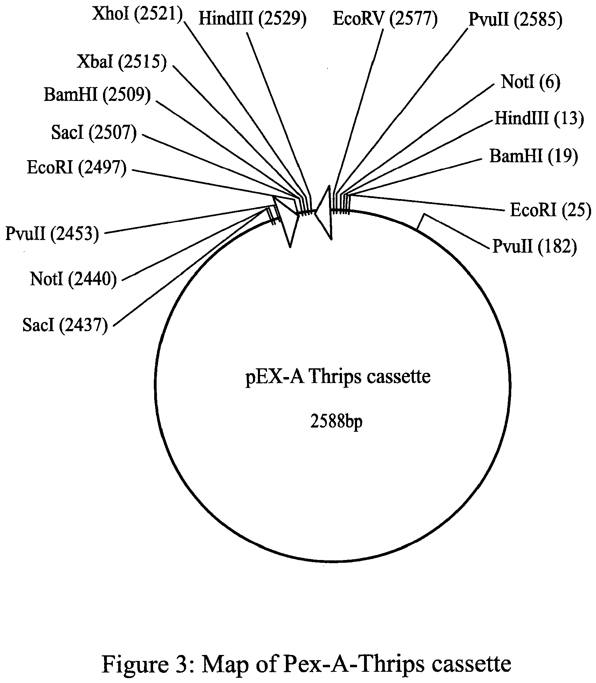

**Figure 3****: shows a Map of the pEX-A-Thrips cassette.**
**Figure 4****: shows plasmid maps of pElong1 and pTub3.** Red arrows indicate *Ptac* promoters and blue section indicates position of dsRNA template sequence.
**Figure 5****: shows plasmid maps of pRN1 and pRN2.** The *rnaseIII* gene is shown in green. Light blue boxes indicate T4 transcription terminators and black arrow indicates position of the apramycin resistance gene.

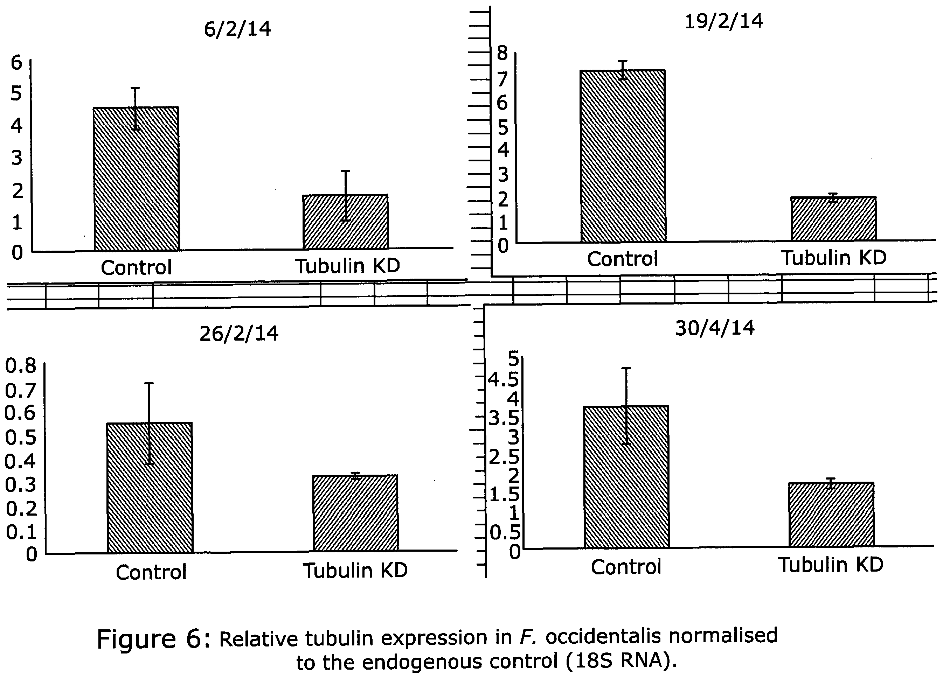

**Figure 6****: shows relative tubulin expression in *F. occidentalis* normalised to the endogenous control (18S RNA).** Bars represent mean relative transcript abundance of tubulin in juvenile insects 48 hours after being fed bacteria (BFo2) expressing double stranded agarose (control) and double stranded tubulin (Tubulin KD). Bars represent mean transcript abundance, error bars represent the standard deviation about the mean. Y axis represent relative tubulin transcript abundance normalised to 18S RNA. Each panel represents a separate experiment using a pool of approximately 22 *F. occidentalis* insects per treatment.

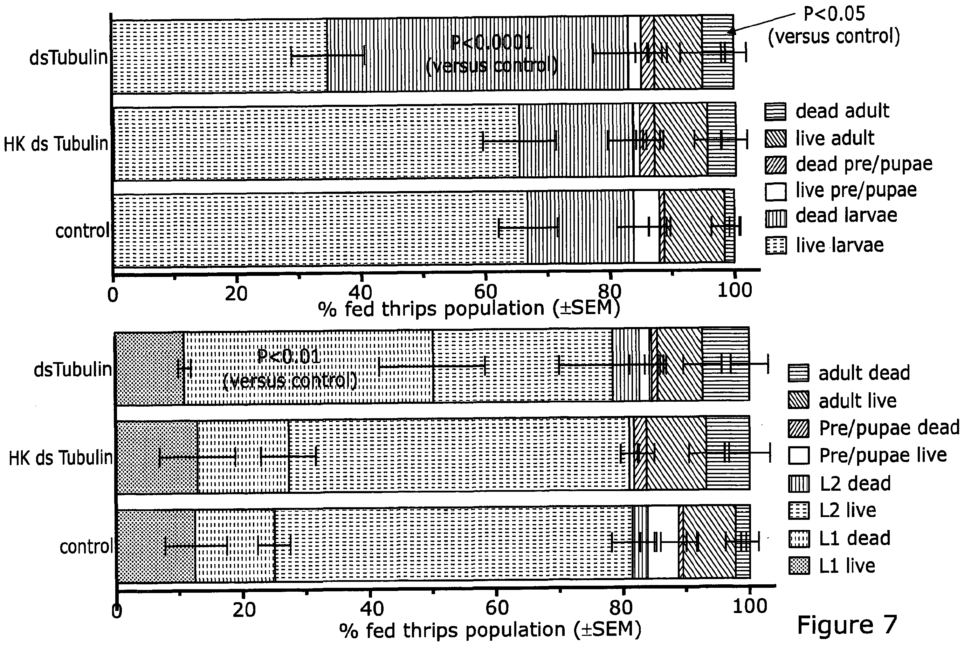

**Figure 7****: shows upper panel: Composition of *F*. *occindentalis* populations following 4 days' oral exposure to modified BFo2 strains expressing control dsRNA or dsTubulin, showing significant a mortality phenotype in the larval and adult stages exposed to dsTubulin.** Data compiled from 7 independent experiments; number of fed insects = 234 (control), 150 (heat-killed [HK] dsTubulin), 220 (dsTubulin). Note: the pre-pupal and pupal stages of thrips are non-feeding. A highly significant tubulin knockdown mortality phenotype was observed among the thrips larvae, and particularly in the first (L1) larval stage. A small but significant mortality was also observed among the adult *F. occidentalis.* Heat-killed (HK) BFo2 expressing dsTubulin failed to elicit the mortality phenotype, which highlights the importance of reintroducing live dsRNA-expressing bacteria as opposed simply to pre-synthesized dsRNA. **Lower panel: Additional experiments identifying the 1^{st} larval stage as most susceptible to the mortality phenotype.** Data compiled from 4 independent experiments; number of fed insects = 134 (control), 95 (HK dsTubulin) & 81 (dsTubulin).

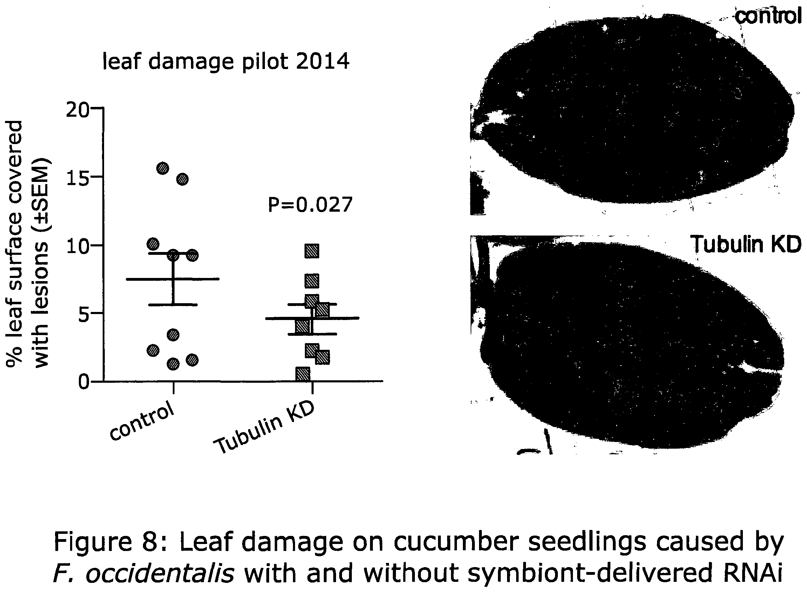

**Figure 8****: shows leaf damage on cucumber seedlings caused by *F. occidentalis* with and without symbiont-delivered RNAi.** Groups of cucumber seedlings were each exposed to WFT larvae and adults that had been orally infected with bacteria (BFo2) expressing dsAgarase RNA (control), or dsTubulin RNA (Tubulin KD). Significantly less damage occurred on plants exposed to *F. occidentalis* receiving the dsTubulin knockdown compared with the controls.

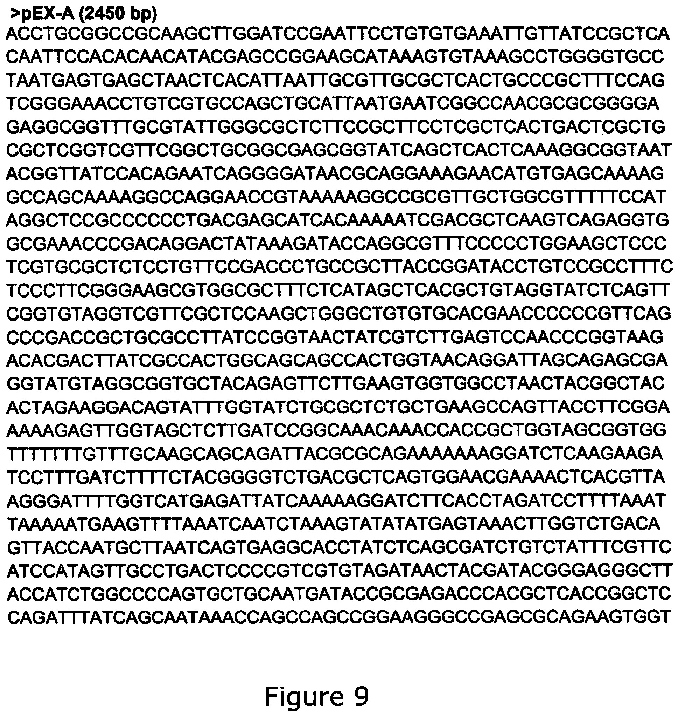

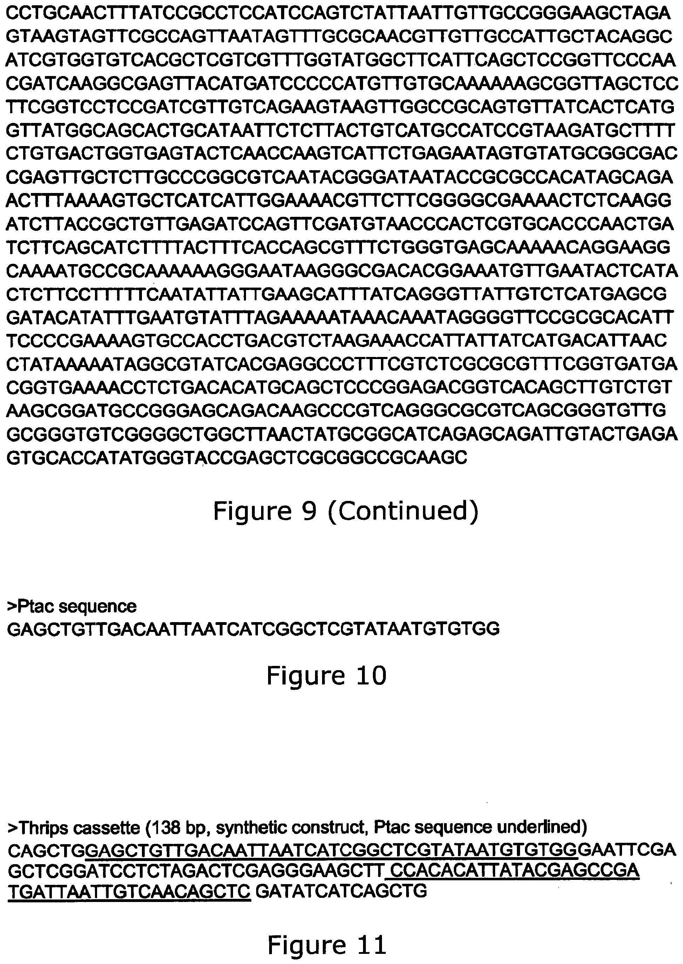

**Figure 9****: shows the sequence structure of the pEX-A (2450 bp) vector.**
**Figure 10****: shows the sequence structure of the Ptac promoter sequence.**
**Figure 11****: shows the sequence structure of the Thrips cassette (138 bp, synthetic construct, Ptac sequence underlined).**

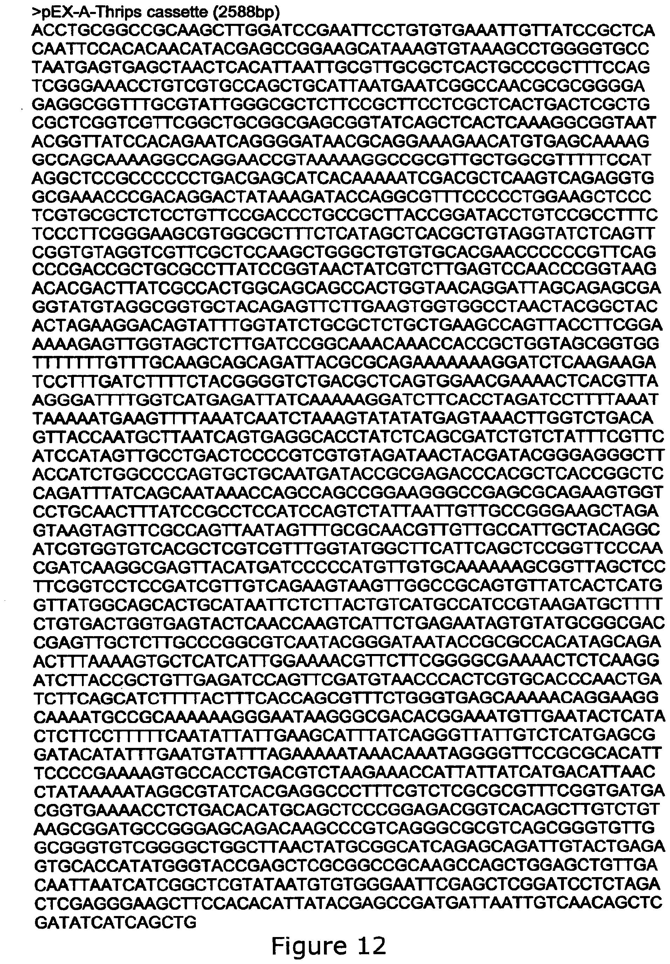

**Figure 12****: shows the sequence structure of the pEX-A-Thrips cassette (2588bp).**
**Figure 13****: shows the sequence structure of the Tubulin alpha-1 chain gene.**
**Figure 14****: shows the sequence structure of the Elongation factor 1-alpha gene.**

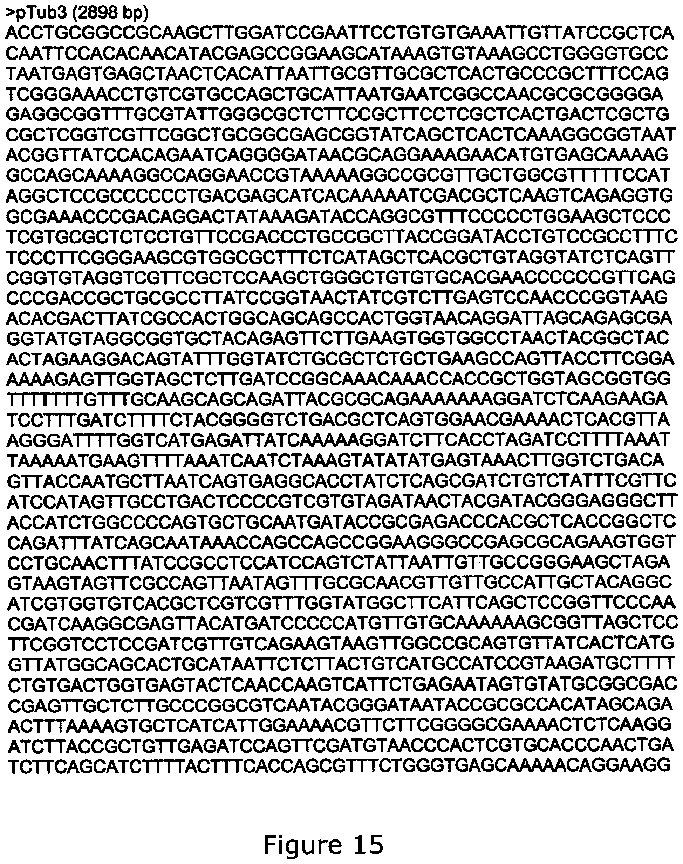

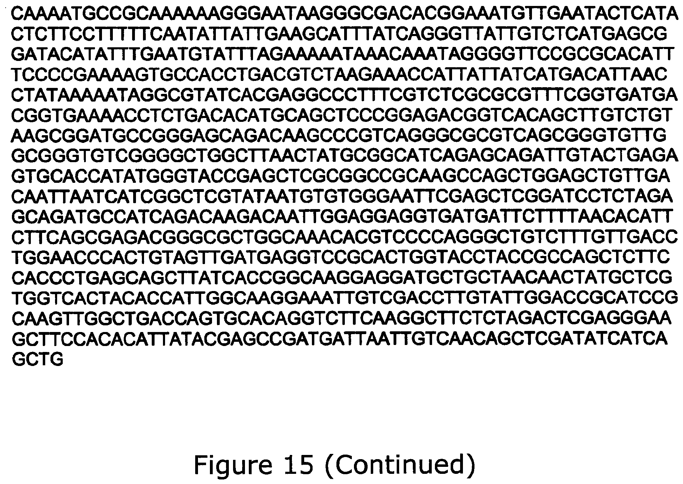

**Figure 15****: shows the sequence structure of the pTub3 plasmid (2898 bp).**

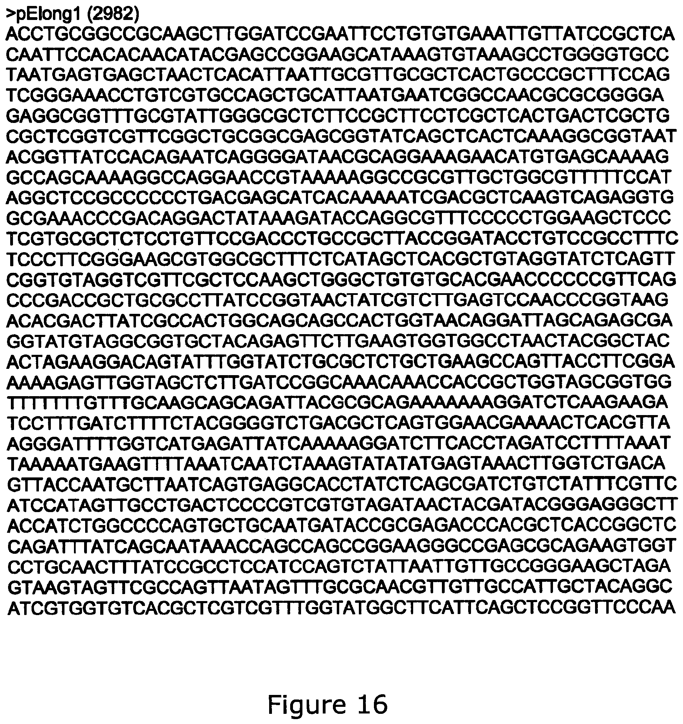

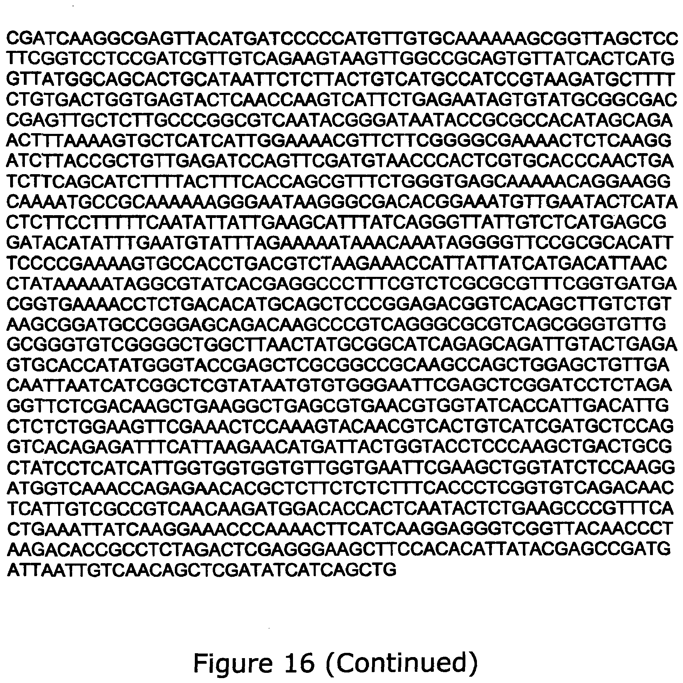

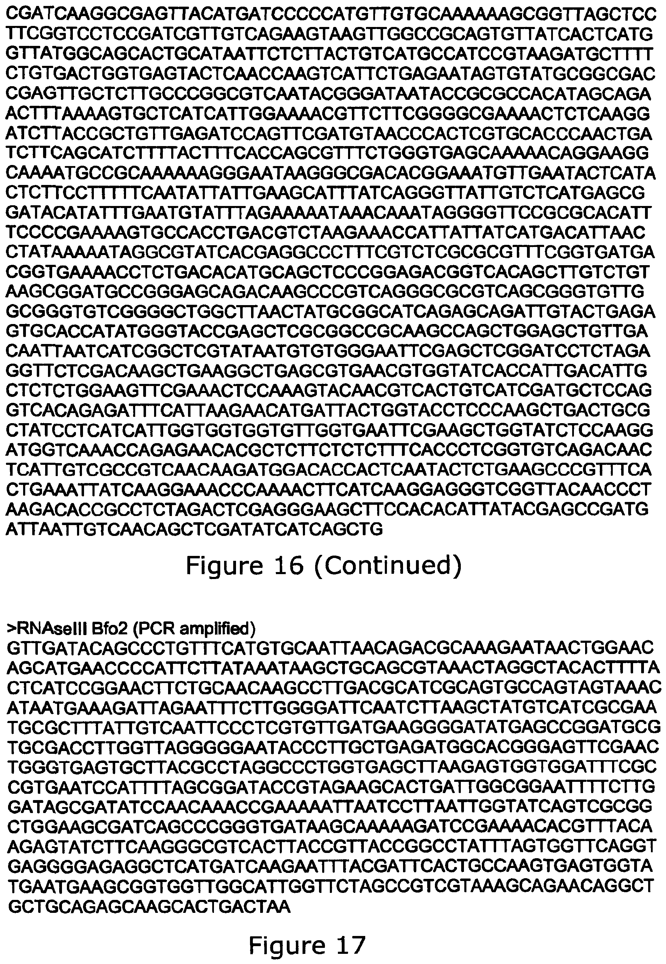

**Figure 16****: shows the sequence structure of the pElong1 plasmid (2982).**
**Figure 17****: shows the sequence structure of RNAseIII Bfo2 (PCR amplified).**

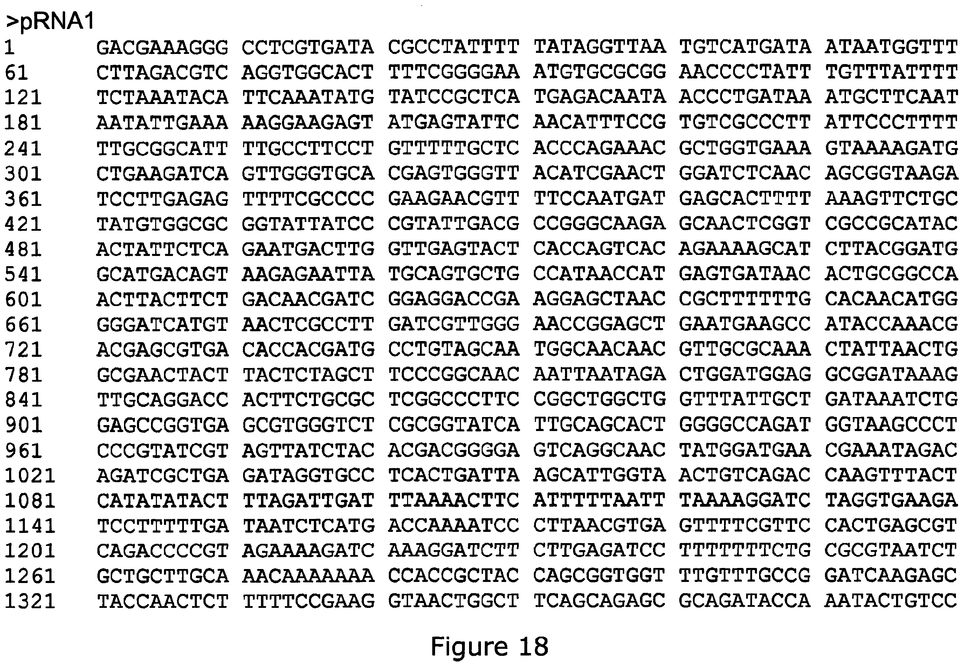

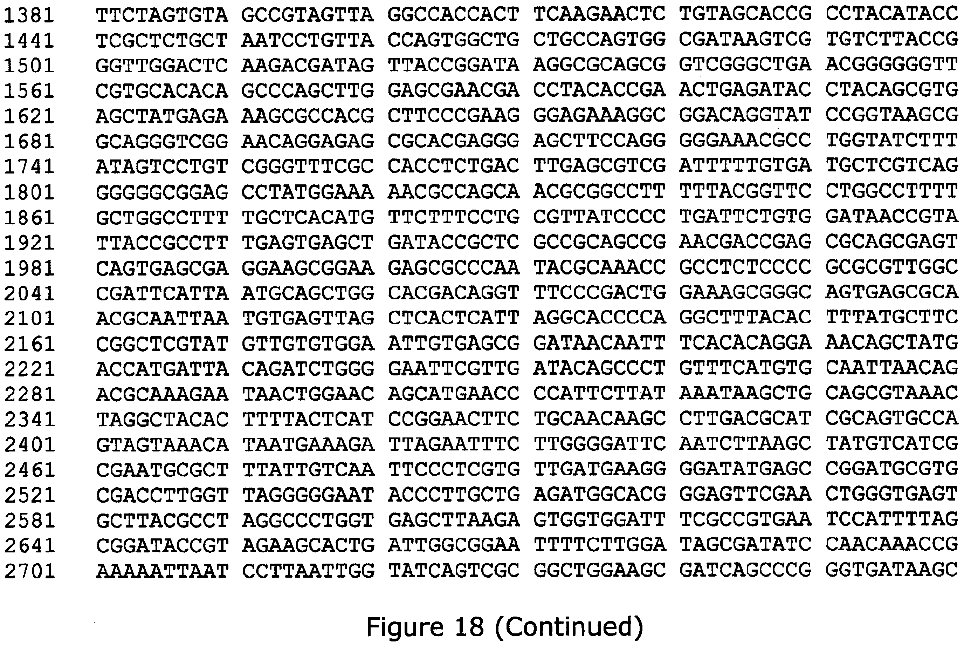

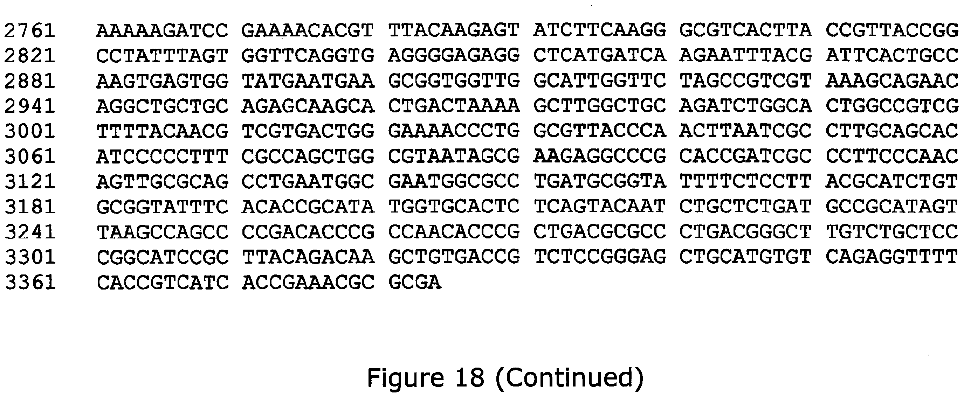

**Figure 18****: shows the sequence structure of pRNA1plasmid.**

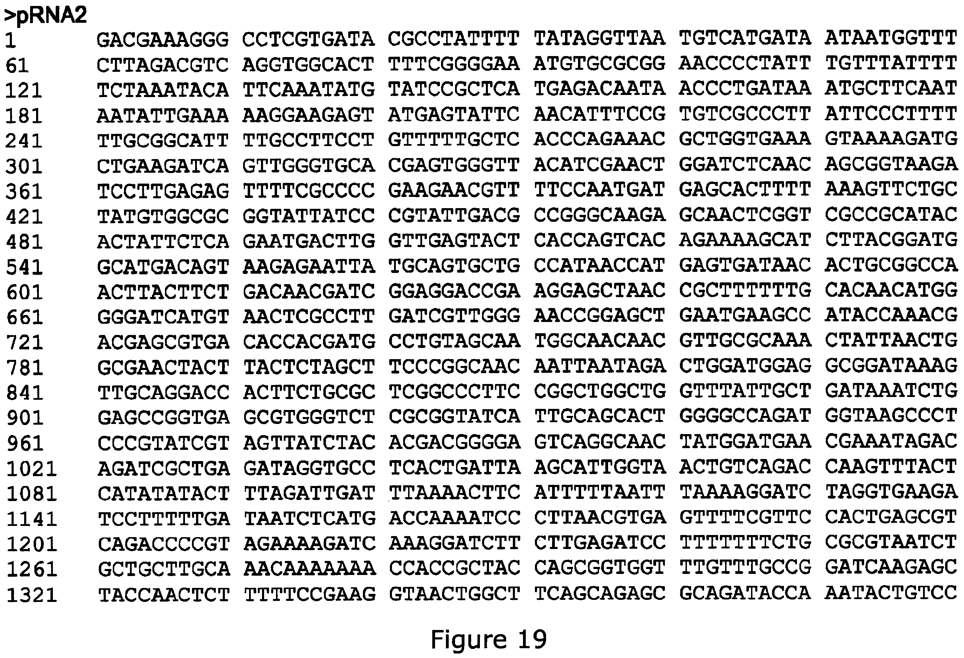

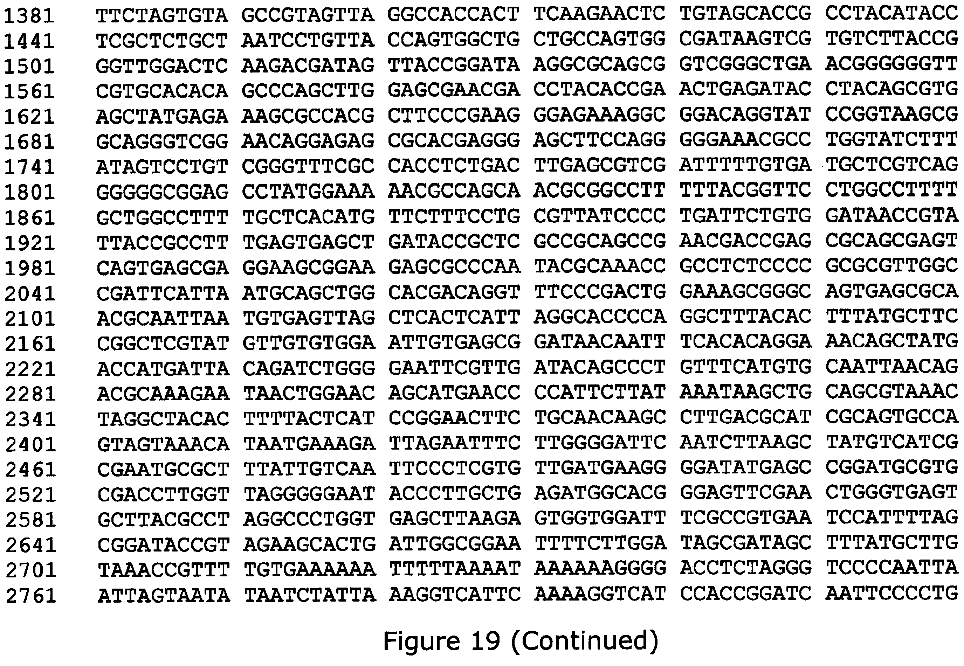

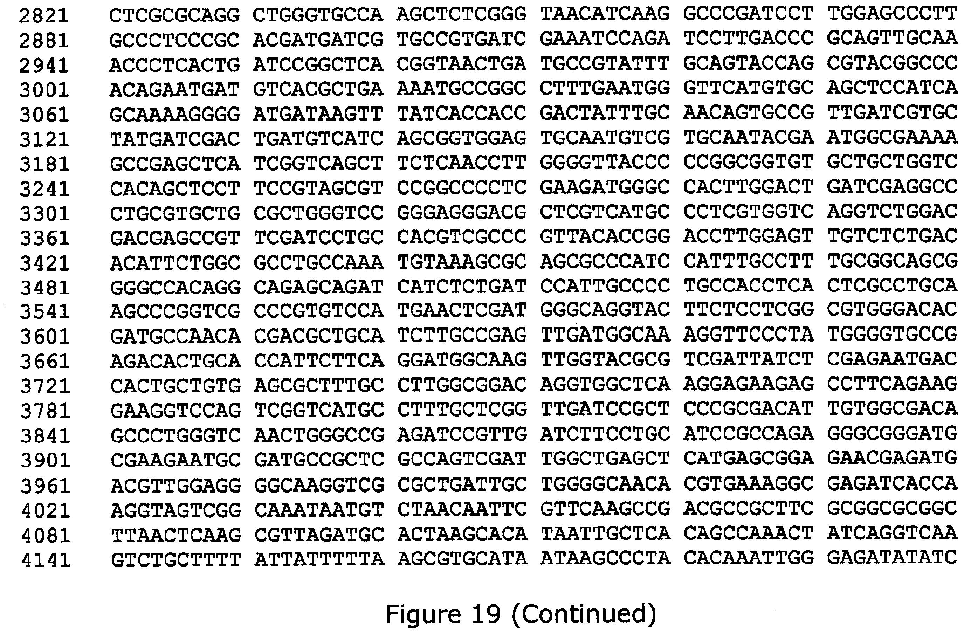

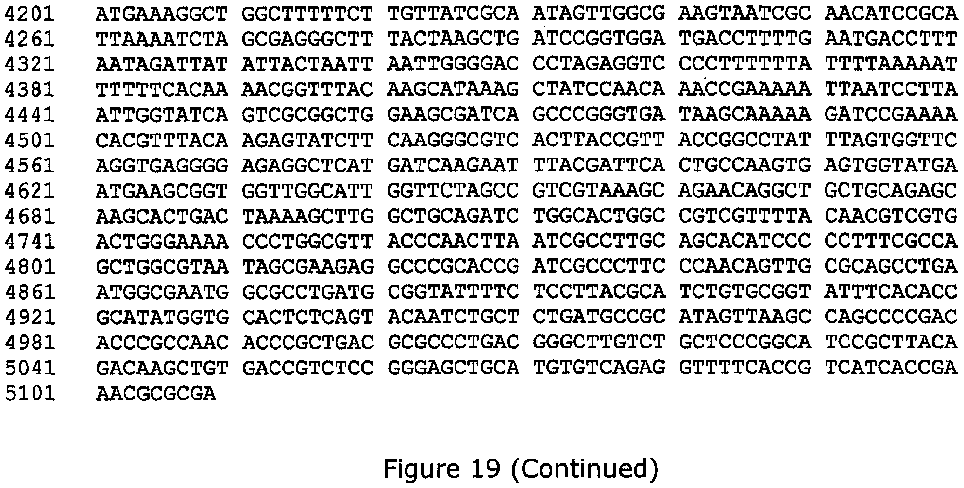

**Figure 19****: shows the sequence structure of pRNA2 plasmid.**

### Example 1

The method involves establishing symbiont-mediated RNAi as a new, robust and tractable means for systemic prolonged gene silencing, suppression or knockdown in WFT, providing a vital research tool to complement the ongoing WFT genome sequencing project (https://www.hgsc.bcm.edu/western-flower-thrips-genome-project) and a potential innovative biocontrol strategy.

WFT contain two species of gut bacteria, one belonging to the genus *Erwinia*, named BFo1, and the second related to the genus *Pantoea,* named BFo2, which can also grow outside the insect host. The bacteria present in the thrips' gut are transmitted to progeny via the leaves that both adults and larvae eat and defaecate on. In second instar larvae, all thrips are infected with the bacteria, and up to 10⁵ bacterial cells are present per thrip. The bacteria attain high populations in larvae, they can be cultured on plates and are tractable for genetic manipulation. The genomes of both BFo1 and BFo2 have been sequenced. Control of transcription is similar to that of E. *coli.*

Stable dsRNA synthesis is dependent on (i) inactivating bacterial RNase III, and (ii) integrating the dsRNA expression cassette into the bacterial genome. This is achieved by engineering the expression cassette into a suitable plasmid and deleting the BFo2 RNase III gene. Recombinants are isolated in which the native RNase III gene is deleted and replaced by an apramycin resistance gene. Plasmids are introduced expressing dsRNA (optimised for RNAi) to target the following WFT genes: tubulin alpha-1 chain (accession number GT305545; GenBank, NCBI); or elongation factor 1-alpha (accession number GT303726; GenBank, NCBI). Knockdown of either of the two target genes severely disables larvae and indicates that the technology is effective against WFT. Stable dsRNA synthesis for each cassette is determined for each recombinant bacterial strain by Q-RTPCR.

Experimental infections were performed in different developmental stages of WFTs by feeding WFTs on recombinant dsRNA-expressing *Erwinia* TAC strains resuspended in an artificial feeding mixture. Thrips were membrane-fed on this mixture as the only food source for 2-4 days. Dye was included in the mixture to non-invasively identify WFTs that had fed. Bacterial growth and viability in the feeding mixture was confirmed at the beginning and end of each experiment by culturing on selective media. The gut contents of WFTs was also cultured on selective media to verify the viability and population of ingested recombinant BFo2 bacteria. Retention of the symbiotic characteristics of the bacteria was assessed by following WFT development in repopulated insects expressing dsAgarase (negative control) and dsTubulin, correlating these measurements with the presence of recombinant bacteria in the gut and by Q-RTPCR of Tubulin mRNA present in RNA preparations from the insects.

Insects populated with BFo2 strains expressing dsRNA targeting the insect tubulin genes were compared with the insects populated with bacteria expressing the negative control dsRNA. Data for insect mortality was determined in each case. These data were correlated with Q-RTPCR assays to measure the abundance of mRNA of the respective target genes.

This novel RNAi strategy can prevent infection of plants by tospoviruses (by killing juvenile insects before they can fly, by targeting vector competence genes of the insect, for example encoding an attachment protein for the virus [Kikkert M., Meurs C., van de Wetering F., Dormüller S., Peters D., Kormelink R., Goldbach R., 1998. Phytopathology 88: 63-69.]and/or viral gene expression), and provide a platform for devising an effective crop protection strategy using the recombinant bacteria as a biopesticide.

Bacteria typically express an enzyme, RNaselll, which specifically degrades dsRNA. Indeed we have established that dsRNA is unstable after it is expressed in BFo2. To circumvent this problem, we have engineered a BFo2 mutant strain in which the gene encoding RNaseIII is disrupted and which stably expresses dsRNA.

### Materials and Procedure

### Bacterial strains and media

Cloning procedures were performed in E. *coli* JM109. Disruption of the *RNaseIII* gene was performed in BFo2 containing pIJ790 to allow Lambda red-mediated recombination (Gust B, Challis GL, Fowler K, Kieser T, Chater KF (2003) Proc Natl Acad Sci U S A. Feb 18;100(4):1541-6.)). Culturing of *E*. *coli* strains was as recommended (Sambrook and Russell (2001). Molecular Cloning: A Laboratory Manual (3rd ed.). Cold Spring Harbor Laboratory Press. ISBN 978-0-87969-577-4). BFo2 was grown at 30°C in liquid culture (LB, with shaking) and on the surface of L agar plates. The identity of the *RNase*III disruption mutant was confirmed by PCR.

### dsRNA expression system

### Construction of expression vector to drive constitutive expression of double stranded RNA in Bfo2.

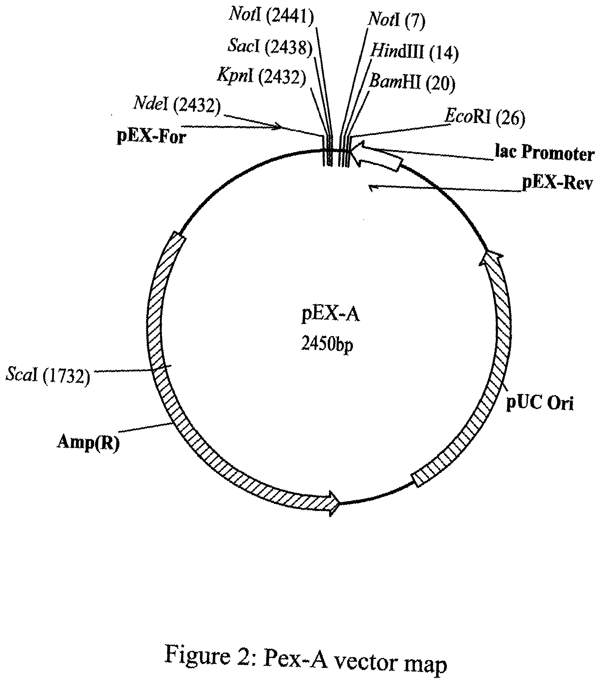

A 138 bp synthetic expression cassette (Eurofins), containing several restriction sites flanked by two copies of the modified constitutive promoter *Ptac* was used. The promoter sequences were designed to drive transcription in a convergent manner (Figure 1), to ensure transcription from both complementary strands of DNA fragments sub-cloned in the MC site. The synthetic expression cassette was cloned between the NotI sites of the ampicillin resistant plasmid pEX-A (Eurofins, Figure 2), generating plasmid pEX-A-Thrips cassette.

Deqor (Henschel et al, 2004, Nucleic Acids Research, 32(Web Server issue):W113-20) was used to help predict *F. occidentalis* genes used as target candidates for double stranded RNA mediated interference. The sequences deemed suitable candidates were obtained by synthesis (Eurofins), flanked by Xbal sites. These DNA fragments were provided as inserts cloned in pEX-A or pCR2 vectors (Table 2).

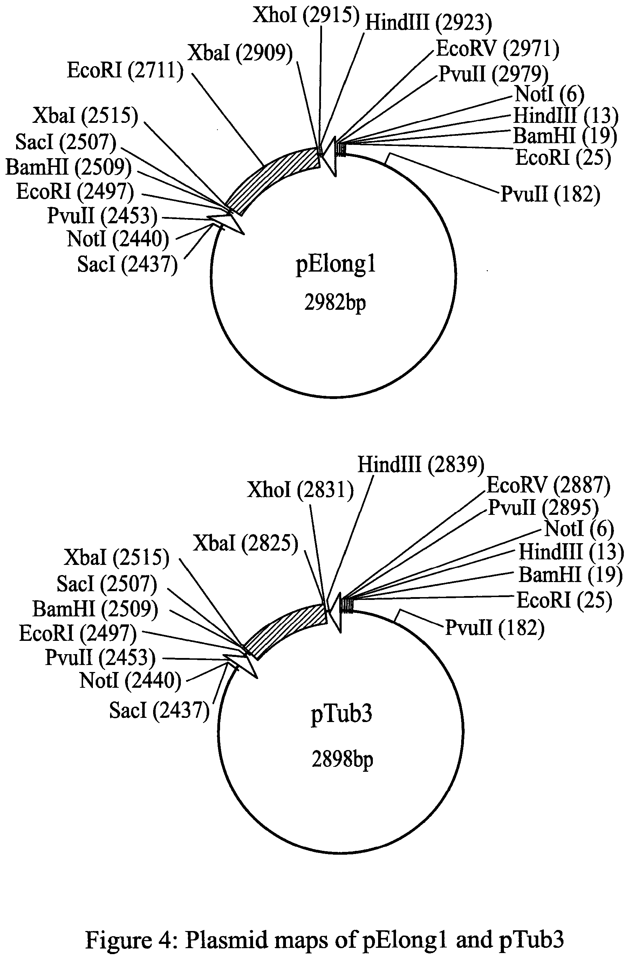

The DNA fragments to be used as templates for dsRNA synthesis were sub-cloned into the Xbal site of pEX-A-Thrips cassette, and therefore flanked by convergent *Ptac* promoters to ensure constitutive transcription of two complementary RNA strands that would hybridise to generate the desired double stranded RNA. The resulting in plasmids were pElong1 and pTub3. These constructs were verified by restriction and DNA sequencing (Figure 4).

### Generation of a BFo2 disruption mutant.

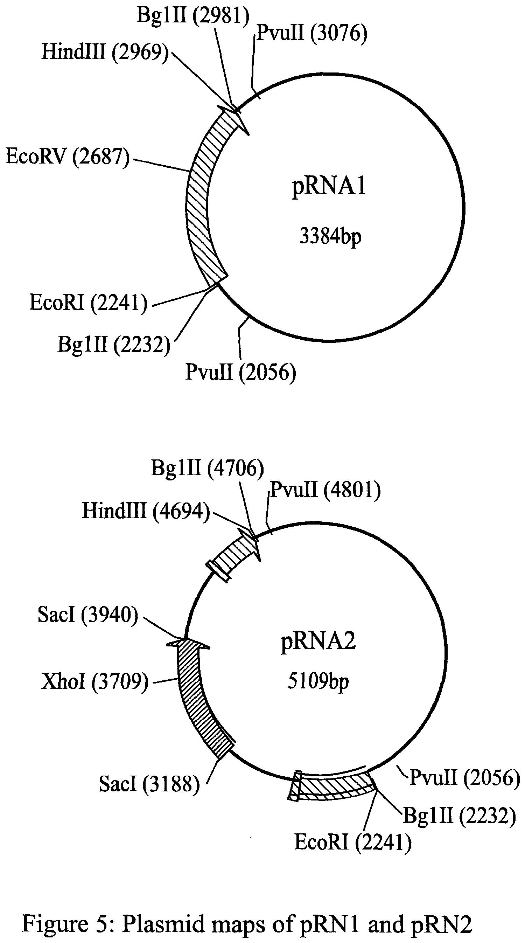

The *RNaseIII* gene was PCR amplified from wild-type BFo2 using primers RIIIBfo2F1 and RIIIBfo2R1 (Table 1). The product (759 bp in length) was digested with EcoRI/HindIII and ligated into pIJ2925 previously digested with EcoRI/HindIII generating plasmid pRNA1. Disruption of this copy of the *RNaseIII* gene was achieved by EcoRV digestion of pRNA1 and insertion of the apramycin resistance gene (flanked by T4 transcription terminators) excised from plasmid pQM5062 by Hindlll restriction digest and blunt ending using T4 DNA polymerase in the presence of 1mM dNTPs giving rise to pRNA2 (Figure 5). To facilitate lambda-red mediated transformation, electrocompetent BFo2 cells were created as recommended for E. *coli* (Sambrook and Russell (2001). Molecular Cloning: A Laboratory Manual (3rd ed.). Cold Spring Harbor Laboratory Press. ISBN 978-0-87969-577-4) and transformed by electroporation with plasmid pIJ790 (Gust B, Challis GL, Fowler K, Kieser T, Chater KF (2003) Proc Natl Acad Sci U S A. Feb 18;100(4):1541-6.)) - creating strain BFo2/pIJ790. The disrupted *RNaseIII* gene was excised from pRNA2 by digesting BgIII and, after gel purification was introduced into electrocompetant BFo2/pIJ790 by electroporation. Double cross-over transformants were selected by replica plating on selective media containing ampicillin or apramycin. Viable colonies were tested for the presence of the disruption by colony PCR using the primers BFo2RNasetestF and BFo2RNasetestR. All plasmids were confirmed by restriction digestion and sequencing.

### Testing functionality of dsRNA expression system

Overnight cultures of BFo2 containing the dsRNA expression plasmids were pelleted by centrifugation (13,000 X g for 10 min) and mixed with equal volumes of RNA protect (Qiagen). Total RNA was isolated using the RNeasy mini RNA isolation kit (Qiagen). Genomic DNA was digested using on-column DNAse I treatment for 45 minutes at room temperature. Strand specific reverse transcription was performed on 1 ug of total RNA using the iscript select reverse transcription kit (Biorad) including the gene specific primer enhancer (GSP) and one of the following gene specific primer pairs: tubulin - TubFoF1 and TubFoR1, elongation factor - EFFoF1 and EFFoR1, (sequences shown in table 3). cDNAs were diluted 1/3 in nuclease free water (supplier). Strand-specific Real Time amplification was performed in triplicate using Sybr-Green Supermix (Biorad) and both gene specific primers listed in table 1. No template controls (NTC, non-reverse transcription) were used to assess the extent of genomic DNA carry over.

### Infection of thrips with BFo2 strains

Overnight cultures of BFo2 containing the dsRNA expression plasmids were pelleted by centrifugation (3,000 X g for 2 min) and washed by resuspension in Luria broth, then resuspended to 5x10⁶/ml in an artificial feeding mixture (20% (v/v) Luria broth, 2.4% (w/v) sucrose, 0.32% (w/v) NaCl and 0.03% (w/v) methylene blue). *Franliniella occidentalis* of all developmental stages were membrane-fed on the feeding mixture as the only food source for 2-4 days from an inverted Bijou bottle reservoir covered by stretched Parafilm. Methylene blue was included to non-invasively identify WFTs that had fed (the blue colour in the gut being visible through the insect cuticle under low-power magnification in WFTs anaesthetised by CO₂). Bacterial growth and viability in the feeding mixture was confirmed at the beginning and end of each experiment by culturing on LB agar supplemented with 1.5% (w/v) sucrose and the appropriate selective antibiotic (apramycin for BFo2 expressing dsAgarase; apramycin and ampicillin for BFo2 expressing dsTubulin). The dyed gut contents of randomly-selected, surface-sterilized WFTs were also cultured on selective media to verify the viability and population of ingested BFo2 strains. Additional controls were prepared using overnight cultures of BFo2 expressing dsTubulin, which were heat-killed by incubation at 65°C for 20 mins prior to incorporation into feeding mixtures as above.

### Efficacy of RNAi in infected insects

Juvenile thrips (1^{st} and 2^{nd} instar) were sampled 48 hours after infection with recombinant BFo2, RNA extracted and levels of tubulin alpha1 mRNA quantified (Figure 6). Total RNA was isolated from pools of approximately 22 juvenile insects using the ZR Tissue and insect RNA microprep kit (Zymo Research). On-column DNase treatment was performed at room temperature using RNase free DNAse (Qiagen). For each isolation, 500 ng of RNA was reverse transcribed using the iscript select synthesis kit and oligodT primers (BioRad). Q RT-PCRs were performed in triplicate using Sybr Green Supermix (BioRad). Serial dilutions of pooled cDNA were performed in duplicate and used to generate standard curves and to test the efficiency of the q RT PCR reaction. Relative transcript abundance of alpha tubulin was calculated by normalising to 18S. All Q RT PCR primers are listed in table 3.

### Mortality of infected insects

*Frankliniella occidentalis* populations were reared on chrysanthemum plants and runner beans *ad libitum* at 70-80% relative humidity, 26-27°C, with a light : dark cycle of 14 : 10 hours respectively. Sample WFT populations containing all developmental stages of *F. occidentalis* were orally infected with recombinant BFo2 expressing dsAgarose RNA (control) and dsTubulin, and monitored for a knockdown phenotype after 4 days. An additional control was included which involved feeding of heat-killed BFo2 expressing dsTubulin.

### Plant protection

Groups of three 15-day-old cucumber seedlings were each exposed to 50 larvae and 15 adult female *F. occidentalis* that had been orally infected with BFo2 expressing dsAgarase RNA (control), or dsTubulin RNA (Tubulin KD). After 5 days, the percentage of the leaf surface that was covered with lesions was assessed by Assess 2.0 image analysis software for plant disease quantification (Lamari, Amer Phytopathological Society; 2008; http://www.apsnet.org/press/assess); (Figure 5).

**Table 1: Strains and plasmids.**

| **Strains** | **genotype/comments** | **Source** |
|---|---|---|
| *E. coli* JM109 | F*'traD36 proA*⁺*B*⁺*lacIq*Δ(*lacZ*)*M15*/Δ (*lac-proAB*) *glnV44 e14- gyrA96 recA1relA1endA1 thi hsdR17* | Yanish-Perron et al., 1985 |
| | | |

| **Plasmids** | | |
|---|---|---|
| pIJ2925 | bla, lacZ | Kieser et al., 2000 |
| pRNA1 | pIJ2925 containing ∼759bp BFo2 rnaselll PCR product | This study |
| pRNA2 | pIJ2925 containing apramycin disrupted BFo2 rnaselll PCR product | This study |
| pIJ790 | λ-RED (gam, bet, exo), cat, araC, rep101ts | Gust et al., 2003 |
| pQM5062 | pMOD+Tn*5062*, Ampicillin^{R} and Apramycin^{R} | Bishop et al., 2004 |

**Table 2: Gene targets used to generate synthetic DNA fragments to be used as template for dsRNA synthesis.**

| Target gene (Genebank) | Plasmid name | Length of synthetic sequence |
|---|---|---|
| Elongation factor 1A (GT303726.1) | pEX-A-Elongation factor 1A | 400 bp |
| Tubulin Alpha-1 chain (GT305545.1) | pEX-A-tubulin alpha-1 | 316 bp |

**Table 3. Oligonucleotides used for PCR and Q RT-PCR.**

| **Oligonucleotides** | |
|---|---|
| RIIIBfo2F1 | TTAGAATTCGTTGATACAGCCCTGTTTCATGTGC |
| RIIIBfo2R1 | TTTAAGCTTTTAGTCAGTGCTTGCTCTGCAGC |
| BFo2RNasetestf | AGC GGA TAC CGT AGA AGC AC |
| BFo2RNasetestR | TAG GCC GGT AAC GGT AAG TG |
| TubFoF1 | AGG ATG CTG CTA ACA ACT A |
| TubFoR1 | GAT GCG GTC CAA TAC AAG |
| EFFoF1 | CTC CAG GTC ACA GAG ATT |
| EFFoR1 | CAC CAA TGA TGA GGA TAG C |
| Fo18SF | GAA GGA TTG ACA GAT TGA |
| Fo18SR | TAG AGT CTC GTT CGT TAT |
| FoTubF | TTG AAG AAG CAT CCT AAC |
| FoTubR | TTG AGA AGT AGT TGA GAT |

## Claims

1. A genetically transformed or transfected bacterial cell wherein said bacteria is a gut symbiont of an insect belonging to the Order *Thysanoptera* **characterised in that** said bacterial cell is transformed or transfected with nucleic acid to express dsRNA against at least a part of tubulin gene or at least a part of elongation factor gene of the insect.

2. The genetically transformed or transfected bacterial cell according to claim 1 wherein said dsRNA is against at least a part of tubulin alpha-1 chain gene or at least a part of elongation factor 1-alpha gene of the insect.

3. The genetically transformed or transfected bacterial cell according to claim 1 or claim 2 wherein said dsRNA comprises a strand of RNA that shares 50% complementarity to at least one of said genes.

4. The genetically transformed or transfected bacterial cell according to claim 3 wherein said dsRNA comprises a strand of RNA that shares at least 75% 80%, 85%, 90%, 91%, 92%, 93%, 94%, 95%, 96%, 97%, 98% or 99% complementarity to at least one of said genes.

5. The genetically transformed or transfected bacterial cell according to claim 3 or 4 wherein said dsRNA active against said tubulin gene or said elongation factor gene of the insect is complementary to at least a part of the sequence structure shown in Figure 13 or 14.

6. The genetically transformed or transfected bacterial cell according to claim 3 or 4 wherein dsRNA comprises a strand of RNA that shares at least 75% 80%, 85%, 90%, 91%, 92%, 93%, 94%, 95%, 96%, 97%, 98% or 99% complementarity to at a part of the sequence structure shown in Figure 13 or 14.

7. The genetically transformed or transfected bacterial cell according to any preceding claim wherein said insect belongs to the *Thripidae family.*

8. The genetically transformed or transfected bacterial cell according to claim 7 wherein said insect belongs to the genus *Frankliniella.*

9. The genetically transformed or transfected bacterial cell according to claim 8 wherein said insect belongs to the species *Frankliniella occidentalis.*

10. The genetically transformed or transfected bacterial cell according to any preceding claim wherein said bacteria is a gut symbiont of the genus *Pantoea;* or an *Erwinia* species gut symbiont.

11. The genetically transformed or transfected bacterial cell according to claim 10 wherein said bacteria is Bacteria, *F. occidentalis* 2 (BFo2) or Bacteria, *F*. *occidentalis* 1 (BFo1).

12. An insect belonging to the Order *Thysanoptera* **characterised in that** said insect comprises a genetically transformed or transfected bacterial cell wherein said bacteria is a gut symbiont of said insect and is transformed to express dsRNA against at least a part of tubulin gene or at least a part of elongation factor gene of the insect.

13. The insect according to claim 12 wherein said dsRNA is against at least a part of tubulin alpha-1 chain gene or at least a part of elongation factor 1-alpha of the insect.

14. Use of the bacterial cell according to any one of claims 1-11 in a method for modulating expression of a target gene of an insect belonging to the Order *Thysanoptera* comprising:
contaminating a composition to be ingested by the insect with said bacterial cell;
whereupon ingestion of said bacterial cell by said insect results in said bacterial cell colonising the gut of said insect wherein it synthesises dsRNA against at least a part of tubulin gene or at least a part of elongation factor gene to modulate said insect gene expression.

15. Use according to claim 14 wherein said dsRNA is against at least a part of tubulin alpha-1 chain gene or at least a part of elongation factor 1-alpha of the insect.

16. A method for modulating the expression of a target gene of an insect belonging to the Order *Thysanoptera* comprising:
contaminating a composition to be ingested by the insect with a bacterial cell according to any one of claims 1-11;
whereupon ingestion of said bacterial cell by said insect results in said bacterial cell colonising the gut of said insect wherein it synthesises dsRNA against at least a part of tubulin gene or at least a part of elongation factor gene to modulate said insect gene expression.

17. The method according to claim 16 wherein said dsRNA is against at least a part of tubulin alpha-1 chain gene or at least a part of elongation factor 1-alpha of the insect.

18. The method according to claim 16 or 17 wherein said bacterial cell is transformed or genetically modified such that recombinant DNA is stably integrated into the host cell genome.

19. The method of claim 18 wherein said nucleic acid or recombinant DNA is stably integrated in the *RNaseIII* gene.

20. The method according to any one of claims 16-19 wherein said modulating the expression of a target gene causes insect death.

21. The method according to any one of claim 20 wherein said death occurs at the larval stage.

22. The method according to any one of claims 23-28 wherein said modulating the expression of a target gene prevents transmission of a pathogenic organism.

23. The method according to claim 22 wherein said pathogenic organism is a tospovirus.

24. The Use according to claims 14-15 or a method according to any one of claims 16-23 wherein said composition comprises a food source for the insect.

25. The Use according to claims 14-15 or a method according to claims 16-23 wherein said composition comprises a plant, faeces or frass.

## Patentansprüche

1. Genetisch transformierte oder transfizierte Bakterienzelle, wobei es sich bei dem Bakterium um einen Darmsymbionten eines Insekts handelt, das der Ordnung *Thysanoptera* angehört, **dadurch gekennzeichnet, dass** die Bakterienzelle mit Nukleinsäure transformiert oder transfiziert ist, um dsRNA gegen mindestens einen Teil des Tubulingens oder mindestens einen Teil des Elongationsfaktorgens des Insekts zu exprimieren.

2. Genetisch transformierte oder transfizierte Bakterienzelle nach Anspruch 1, wobei die dsRNA gegen mindestens einen Teil des Gens für die Tubulin-alpha-1-Kette oder mindestens einen Teil des Elongationsfaktor-1-alpha-Gens des Insekts gerichtet ist.

3. Genetisch transformierte oder transfizierte Bakterienzelle nach Anspruch 1 oder Anspruch 2, wobei die dsRNA einen RNA-Strang umfasst, der 50 % Komplementarität mit mindestens einem der Gene gemeinsam hat.

4. Genetisch transformierte oder transfizierte Bakterienzelle nach Anspruch 3, wobei die dsRNA einen RNA-Strang umfasst, der mindestens 75 %, 80 %, 85 %, 90 %, 91 %, 92 %, 93 %, 94 %, 95 %, 96 %, 97 %, 98 % oder 99 % Komplementarität mit mindestens einem der Gene gemeinsam hat.

5. Genetisch transformierte oder transfizierte Bakterienzelle nach Anspruch 3 oder 4, wobei die dsRNA, die gegen das Tubulingen oder das Elongationsfaktorgen des Insekts aktiv ist, zu mindestens einem Teil der in Figur 13 oder 14 dargestellten Sequenzstruktur komplementär ist.

6. Genetisch transformierte oder transfizierte Bakterienzelle nach Anspruch 3 oder 4, wobei die dsRNA einen RNA-Strang umfasst, der mindestens 75 %, 80 %, 85 %, 90 %, 91 %, 92 %, 93 %, 94 %, 95 %, 96 %, 97 %, 98 % oder 99 % zu mindestens einem Teil der in Figur 13 oder 14 dargestellten Sequenzstruktur komplementär ist.

7. Genetisch transformierte oder transfizierte Bakterienzelle nach einem vorhergehenden Anspruch, wobei das Insekt der Familie *Thripidae* angehört.

8. Genetisch transformierte oder transfizierte Bakterienzelle nach Anspruch 7, wobei das Insekt der Gattung *Frankliniella* angehört.

9. Genetisch transformierte oder transfizierte Bakterienzelle nach Anspruch 8, wobei das Insekt der Art *Frankliniella occidentalis* angehört.

10. Genetisch transformierte oder transfizierte Bakterienzelle nach einem vorhergehenden Anspruch, wobei es sich bei dem Bakterium um einen Darmsymbionten der Gattung *Pantoea* oder einen Darmsymbionten einer *Erwinia*-Art handelt.

11. Genetisch transformierte oder transfizierte Bakterienzelle nach Anspruch 10, wobei es sich bei dem Bakterium um Bakterium, *F*. *occidentalis* 2 (BFo2) oder Bakterium, *F*. *occidentalis* 1 (BFo1) handelt.

12. Insekt, das der Ordnung *Thysanoptera* angehört, **dadurch gekennzeichnet, dass** das Insekt eine genetisch transformierte oder transfizierte Bakterienzelle umfasst, wobei es sich bei dem Bakterium um einen Darmsymbionten des Insekts handelt und es derart transformiert ist, dass es dsRNA gegen mindestens einen Teil des Tubulingens oder mindestens einen Teil des Elongationsfaktorgens des Insekts exprimiert.

13. Insekt nach Anspruch 12, wobei die dsRNA gegen mindestens einen Teil des Gens für die Tubulin-alpha-1-Kette oder mindestens einen Teil des Elongationsfaktor-1-alpha-Gens des Insekts gerichtet ist.

14. Verwendung der Bakterienzelle nach einem der Ansprüche 1-11 bei einem Verfahren zum Modulieren der Expression eines Zielgens eines Insekts, das der Ordnung *Thysanoptera* angehört, umfassend:
Verunreinigen einer Zusammensetzung, die von dem Insekt aufzunehmen ist, mit der Bakterienzelle;
woraufhin die Aufnahme der Bakterienzelle durch das Insekt zur Folge hat, dass sich die Bakterienzelle im Darm des Insekts ansiedelt, wobei sie dsRNA gegen mindestens einen Teil des Tubulingens oder mindestens einen Teil des Elongationsfaktorgens synthetisiert, um die Genexpression des Insekts zu modulieren.

15. Verwendung nach Anspruch 14, wobei die dsRNA gegen mindestens einen Teil des Gens für die Tubulin-alpha-1-Kette oder mindestens einen Teil des Elongationsfaktor-1-alpha-Gens des Insekts gerichtet ist.

16. Verfahren zum Modulieren der Expression eines Zielgens eines Insekts, das der Ordnung *Thysanoptera* angehört, umfassend:
Verunreinigen einer Zusammensetzung, die von dem Insekt aufzunehmen ist, mit einer Bakterienzelle nach einem der Ansprüche 1-11;
woraufhin die Aufnahme der Bakterienzelle durch das Insekt zur Folge hat, dass sich die Bakterienzelle im Darm des Insekts ansiedelt, wobei sie dsRNA gegen mindestens einen Teil des Tubulingens oder mindestens einen Teil des Elongationsfaktorgens synthetisiert, um die Genexpression des Insekts zu modulieren.

17. Verfahren nach Anspruch 16, wobei die dsRNA gegen mindestens einen Teil des Gens für die Tubulin-alpha-1-Kette oder mindestens einen Teil des Elongationsfaktor-1-alpha-Gens des Insekts gerichtet ist.

18. Verfahren nach Anspruch 16 oder 17, wobei die Bakterienzelle derart transformiert oder genetisch modifiziert ist, dass rekombinante DNA stabil in das Wirtszellgenom eingebaut wird.

19. Verfahren nach Anspruch 18, wobei die Nukleinsäure oder die rekombinante DNA stabil in das *RNaseIII*-Gen eingebaut wird.

20. Verfahren nach einem der Ansprüche 16-19, wobei das Modulieren der Expression eines Zielgens den Tod des Insekts herbeiführt.

21. Verfahren nach einem von Anspruch 20, wobei der Tod im Larvenstadium eintritt.

22. Verfahren nach einem der Ansprüche 23-28, wobei das Modulieren der Expression eines Zielgens die Übertragung eines Krankheitserregers verhindert.

23. Verfahren nach Anspruch 22, wobei es sich bei dem Krankheitserreger um ein Tospovirus handelt.

24. Verwendung nach den Ansprüchen 14-15 oder Verfahren nach einem der Ansprüche 16-23, wobei die Zusammensetzung eine Nahrungsquelle für das Insekt umfasst.

25. Verwendung nach den Ansprüchen 14-15 oder Verfahren nach einem der Ansprüche 16-23, wobei die Zusammensetzung eine Pflanze, Kot oder Insektenkot umfasst.

## Revendications

1. Cellule bactérienne génétiquement transformée ou transfectée dans laquelle ladite bactérie est un symbiote intestinal d'un insecte appartenant à l'ordre *Thysanoptera* **caractérisée en ce que** ladite cellule bactérienne est transformée ou transfectée avec un acide nucléique pour exprimer un ARNdb contre au moins une partie d'un gène de tubuline ou au moins une partie d'un gène de facteur d'élongation de l'insecte.

2. Cellule bactérienne génétiquement transformée ou transfectée selon la revendication 1 dans laquelle ledit ARNdb est contre au moins une partie d'un gène de chaîne de tubuline alpha-1 ou au moins une partie d'un gène de facteur d'élongation 1-alpha de l'insecte.

3. Cellule bactérienne génétiquement transformée ou transfectée selon la revendication 1 ou la revendication 2 dans laquelle ledit ARNdb comprend un brin d'ARN qui partage une complémentarité de 50 % avec au moins l'un desdits gènes.

4. Cellule bactérienne génétiquement transformée ou transfectée selon la revendication 3 dans laquelle ledit ARNdb comprend un brin d'ARN qui partage une complémentarité d'au moins 75 %, 80 %, 85 %, 90%, 91 %, 92 %, 93 %, 94 %, 95 %, 96 %, 97 %, 98 % ou 99 % avec au moins l'un desdits gènes.

5. Cellule bactérienne génétiquement transformée ou transfectée selon la revendication 3 ou 4 dans laquelle ledit ARNdb actif contre ledit gène de tubuline ou ledit gène de facteur d'élongation de l'insecte est complémentaire avec au moins une partie de la structure de séquence représentée sur la figure 13 ou 14.

6. Cellule bactérienne génétiquement transformée ou transfectée selon la revendication 3 ou 4 dans laquelle l'ARNdb comprend un brin d'ARN qui partage une complémentarité d'au moins 75 %, 80 %, 85 %, 90%, 91 %, 92 %, 93 %, 94 %, 95 %, 96 %, 97 %, 98 % ou 99 % avec une partie de la structure de séquence représentée sur la figure 13 ou 14.

7. Cellule bactérienne génétiquement transformée ou transfectée selon une quelconque revendication précédente dans laquelle ledit insecte appartient à la famille des *Thripidae.*

8. Cellule bactérienne génétiquement transformée ou transfectée selon la revendication 7 dans laquelle ledit insecte appartient au genre *Frankliniella.*

9. Cellule bactérienne génétiquement transformée ou transfectée selon la revendication 8 dans laquelle ledit insecte appartient à l'espèce *Frankliniella occidentalis.*

10. Cellule bactérienne génétiquement transformée ou transfectée selon une quelconque revendication précédente dans laquelle ladite bactérie est un symbiote intestinal du genre *Pantoea* ; ou un symbiote intestinal de l'espèce *Erwinia*.

11. Cellule bactérienne génétiquement transformée ou transfectée selon la revendication 10 dans laquelle ladite bactérie est une bactérie, *F*. *occidentalis* 2 (BFo2) ou une bactérie, *F*. *occidentalis* 1 (BFo1).

12. Insecte appartenant à l'ordre *Thysanoptera* **caractérisé en ce que** ledit insecte comprend une cellule bactérienne génétiquement transformée ou transfectée dans lequel ladite bactérie est un symbiote intestinal dudit insecte et est transformée pour exprimer un ARNdb contre au moins une partie d'un gène de tubuline ou au moins une partie d'un gène de facteur d'élongation de l'insecte.

13. Insecte selon la revendication 12 dans lequel ledit ARNdb est contre au moins une partie d'un gène de chaîne de tubuline alpha-1 ou au moins une partie d'un facteur d'élongation 1-alpha de l'insecte.

14. Utilisation de la cellule bactérienne selon l'une quelconque des revendications 1 à 11 dans un procédé pour moduler l'expression d'un gène cible d'un insecte appartenant à l'ordre *Thysanoptera* comprenant :
la contamination d'une composition à ingérer par l'insecte avec ladite cellule bactérienne ;
après quoi l'ingestion de ladite cellule bactérienne par ledit insecte entraîne la colonisation par ladite cellule bactérienne de l'intestin dudit insecte dans lequel il synthétise un ARNdb contre au moins une partie d'un gène de tubuline ou au moins une partie d'un gène de facteur d'élongation pour moduler ladite expression génétique de l'insecte.

15. Utilisation selon la revendication 14 dans laquelle ledit ARNdb est contre au moins une partie d'un gène de chaîne de tubuline alpha-1 ou au moins une partie d'un facteur d'élongation 1-alpha de l'insecte.

16. Procédé pour moduler l'expression d'un gène cible d'un insecte appartenant à l'ordre *Thysanoptera* comprenant :
la contamination d'une composition à ingérer par l'insecte avec une cellule bactérienne selon l'une quelconque des revendications 1 à 11 ;
après quoi l'ingestion de ladite cellule bactérienne par ledit insecte entraîne la colonisation par ladite cellule bactérienne de l'intestin dudit insecte dans lequel il synthétise un ARNdb contre au moins une partie d'un gène de tubuline ou au moins une partie d'un gène de facteur d'élongation pour moduler ladite expression génétique de l'insecte.

17. Procédé selon la revendication 16 dans lequel ledit ARNdb est contre au moins une partie d'un gène de chaîne de tubuline alpha-1 ou au moins une partie d'un facteur d'élongation 1-alpha de l'insecte.

18. Procédé selon la revendication 16 ou 17 dans lequel ladite cellule bactérienne est transformée ou génétiquement modifiée de façon qu'un ADN recombinant soit intégré de manière stable dans le génome de la cellule hôte.

19. Procédé selon la revendication 18 dans lequel ledit acide nucléique ou ledit ADN recombinant est intégré de manière stable dans le gène *RNAseIII*.

20. Procédé selon l'une quelconque des revendications 16 à 19 dans lequel ladite modulation de l'expression d'un gène cible provoque la mort de l'insecte.

21. Procédé selon la revendication 20 dans lequel ladite mort se produit au stade larvaire.

22. Procédé selon l'une quelconque des revendications 23 à 28 dans lequel ladite modulation de l'expression d'un gène cible empêche la transmission d'un organisme pathogène.

23. Procédé selon la revendication 22 dans lequel ledit organisme pathogène est un tospovirus.

24. Utilisation selon les revendications 14 et 15 ou procédé selon l'une quelconque des revendications 16 à 23 dans lequel ladite composition comprend une source de nourriture pour l'insecte.

25. Utilisation selon les revendications 14 et 15 ou procédé selon les revendications 16 à 23 dans lequel ladite composition comprend une plante, des fèces ou des excréments.


### SEQUENCE LISTING

<110> Swansea University
   Dyson, Paul
   Whitten, Miranda
<120> Pest Control System
<130> 0179AP/WO
<150> GB1510154.6
   <151> 2015-06-11
<160> 23
<170> PatentIn version 3.5
<210> 1
   <211> 34
   <212> DNA
   <213> Frankliniella occidentalis
<400> 1
   ttagaattcg ttgatacagc cctgtttcat gtgc 34
<210> 2
   <211> 32
   <212> DNA
   <213> Frankliniella occidentalis
<400> 2
   tttaagcttt tagtcagtgc ttgctctgca gc 32
<210> 3
   <211> 20
   <212> DNA
   <213> Frankliniella occidentalis
<400> 3
   agcggatacc gtagaagcac 20
<210> 4
   <211> 20
   <212> DNA
   <213> Frankliniella occidentalis
<400> 4
   taggccggta acggtaagtg 20
<210> 5
   <211> 19
   <212> DNA
   <213> Frankliniella occidentalis
<400> 5
   aggatgctgc taacaacta 19
<210> 6
   <211> 18
   <212> DNA
   <213> Frankliniella occidentalis
<400> 6
   gatgcggtcc aatacaag 18
<210> 7
   <211> 18
   <212> DNA
   <213> Frankliniella occidentalis
<400> 7
   ctccaggtca cagagatt 18
<210> 8
   <211> 19
   <212> DNA
   <213> Frankliniella occidentalis
<400> 8
   caccaatgat gaggatagc 19
<210> 9
   <211> 18
   <212> DNA
   <213> Frankliniella occidentalis
<400> 9
   gaaggattga cagattga 18
<210> 10
   <211> 18
   <212> DNA
   <213> Frankliniella occidentalis
<400> 10
   tagagtctcg ttcgttat 18
<210> 11
   <211> 18
   <212> DNA
   <213> Frankliniella occidentalis
<400> 11
   ttgaagaagc atcctaac 18
<210> 12
   <211> 18
   <212> DNA
   <213> Frankliniella occidentalis
<400> 12
   ttgagaagta gttgagat 18
<210> 13
   <211> 2450
   <212> DNA
   <213> Escherichia coli
<400> 13
   acctgcggcc gcaagcttgg atccgaattc ctgtgtgaaa ttgttatccg ctcacaattc 60
   cacacaacat acgagccgga agcataaagt gtaaagcctg gggtgcctaa tgagtgagct 120
   aactcacatt aattgcgttg cgctcactgc ccgctttcca gtcgggaaac ctgtcgtgcc 180
   agctgcatta atgaatcggc caacgcgcgg ggagaggcgg tttgcgtatt gggcgctctt 240
   ccgcttcctc gctcactgac tcgctgcgct cggtcgttcg gctgcggcga gcggtatcag 300
   ctcactcaaa ggcggtaata cggttatcca cagaatcagg ggataacgca ggaaagaaca 360
   tgtgagcaaa aggccagcaa aaggccagga accgtaaaaa ggccgcgttg ctggcgtttt 420
   tccataggct ccgcccccct gacgagcatc acaaaaatcg acgctcaagt cagaggtggc 480
   gaaacccgac aggactataa agataccagg cgtttccccc tggaagctcc ctcgtgcgct 540
   ctcctgttcc gaccctgccg cttaccggat acctgtccgc ctttctccct tcgggaagcg 600
   tggcgctttc tcatagctca cgctgtaggt atctcagttc ggtgtaggtc gttcgctcca 660
   agctgggctg tgtgcacgaa ccccccgttc agcccgaccg ctgcgcctta tccggtaact 720
   atcgtcttga gtccaacccg gtaagacacg acttatcgcc actggcagca gccactggta 780
   acaggattag cagagcgagg tatgtaggcg gtgctacaga gttcttgaag tggtggccta 840
   actacggcta cactagaagg acagtatttg gtatctgcgc tctgctgaag ccagttacct 900
   tcggaaaaag agttggtagc tcttgatccg gcaaacaaac caccgctggt agcggtggtt 960
   tttttgtttg caagcagcag attacgcgca gaaaaaaagg atctcaagaa gatcctttga 1020
   tcttttctac ggggtctgac gctcagtgga acgaaaactc acgttaaggg attttggtca 1080
   tgagattatc aaaaaggatc ttcacctaga tccttttaaa ttaaaaatga agttttaaat 1140
   caatctaaag tatatatgag taaacttggt ctgacagtta ccaatgctta atcagtgagg 1200
   cacctatctc agcgatctgt ctatttcgtt catccatagt tgcctgactc cccgtcgtgt 1260
   agataactac gatacgggag ggcttaccat ctggccccag tgctgcaatg ataccgcgag 1320
   acccacgctc accggctcca gatttatcag caataaacca gccagccgga agggccgagc 1380
   gcagaagtgg tcctgcaact ttatccgcct ccatccagtc tattaattgt tgccgggaag 1440
   ctagagtaag tagttcgcca gttaatagtt tgcgcaacgt tgttgccatt gctacaggca 1500
   tcgtggtgtc acgctcgtcg tttggtatgg cttcattcag ctccggttcc caacgatcaa 1560
   ggcgagttac atgatccccc atgttgtgca aaaaagcggt tagctccttc ggtcctccga 1620
   tcgttgtcag aagtaagttg gccgcagtgt tatcactcat ggttatggca gcactgcata 1680
   attctcttac tgtcatgcca tccgtaagat gcttttctgt gactggtgag tactcaacca 1740
   agtcattctg agaatagtgt atgcggcgac cgagttgctc ttgcccggcg tcaatacggg 1800
   ataataccgc gccacatagc agaactttaa aagtgctcat cattggaaaa cgttcttcgg 1860
   ggcgaaaact ctcaaggatc ttaccgctgt tgagatccag ttcgatgtaa cccactcgtg 1920
   cacccaactg atcttcagca tcttttactt tcaccagcgt ttctgggtga gcaaaaacag 1980
   gaaggcaaaa tgccgcaaaa aagggaataa gggcgacacg gaaatgttga atactcatac 2040
   tcttcctttt tcaatattat tgaagcattt atcagggtta ttgtctcatg agcggataca 2100
   tatttgaatg tatttagaaa aataaacaaa taggggttcc gcgcacattt ccccgaaaag 2160
   tgccacctga cgtctaagaa accattatta tcatgacatt aacctataaa aataggcgta 2220
   tcacgaggcc ctttcgtctc gcgcgtttcg gtgatgacgg tgaaaacctc tgacacatgc 2280
   agctcccgga gacggtcaca gcttgtctgt aagcggatgc cgggagcaga caagcccgtc 2340
   agggcgcgtc agcgggtgtt ggcgggtgtc ggggctggct taactatgcg gcatcagagc 2400
   agattgtact gagagtgcac catatgggta ccgagctcgc ggccgcaagc 2450
<210> 14
   <211> 40
   <212> DNA
   <213> Escherichia coli
<400> 14
   gagctgttga caattaatca tcggctcgta taatgtgtgg 40
<210> 15
   <211> 138
   <212> DNA
   <213> Artificial Sequence
<220>
   <223> thrips cassette comprising PTAC sequence
<400> 15
   cagctggagc tgttgacaat taatcatcgg ctcgtataat gtgtgggaat tcgagctcgg 60
   atcctctaga ctcgagggaa gcttccacac attatacgag ccgatgatta attgtcaaca 120
   gctcgatatc atcagctg 138
<210> 16
   <211> 2588
   <212> DNA
   <213> Artificial Sequence
<220>
   <223> pEX-A-Thrips Cassette
<400> 16
   acctgcggcc gcaagcttgg atccgaattc ctgtgtgaaa ttgttatccg ctcacaattc 60
   cacacaacat acgagccgga agcataaagt gtaaagcctg gggtgcctaa tgagtgagct 120
   aactcacatt aattgcgttg cgctcactgc ccgctttcca gtcgggaaac ctgtcgtgcc 180
   agctgcatta atgaatcggc caacgcgcgg ggagaggcgg tttgcgtatt gggcgctctt 240
   ccgcttcctc gctcactgac tcgctgcgct cggtcgttcg gctgcggcga gcggtatcag 300
   ctcactcaaa ggcggtaata cggttatcca cagaatcagg ggataacgca ggaaagaaca 360
   tgtgagcaaa aggccagcaa aaggccagga accgtaaaaa ggccgcgttg ctggcgtttt 420
   tccataggct ccgcccccct gacgagcatc acaaaaatcg acgctcaagt cagaggtggc 480
   gaaacccgac aggactataa agataccagg cgtttccccc tggaagctcc ctcgtgcgct 540
   ctcctgttcc gaccctgccg cttaccggat acctgtccgc ctttctccct tcgggaagcg 600
   tggcgctttc tcatagctca cgctgtaggt atctcagttc ggtgtaggtc gttcgctcca 660
   agctgggctg tgtgcacgaa ccccccgttc agcccgaccg ctgcgcctta tccggtaact 720
   atcgtcttga gtccaacccg gtaagacacg acttatcgcc actggcagca gccactggta 780
   acaggattag cagagcgagg tatgtaggcg gtgctacaga gttcttgaag tggtggccta 840
   actacggcta cactagaagg acagtatttg gtatctgcgc tctgctgaag ccagttacct 900
   tcggaaaaag agttggtagc tcttgatccg gcaaacaaac caccgctggt agcggtggtt 960
   tttttgtttg caagcagcag attacgcgca gaaaaaaagg atctcaagaa gatcctttga 1020
   tcttttctac ggggtctgac gctcagtgga acgaaaactc acgttaaggg attttggtca 1080
   tgagattatc aaaaaggatc ttcacctaga tccttttaaa ttaaaaatga agttttaaat 1140
   caatctaaag tatatatgag taaacttggt ctgacagtta ccaatgctta atcagtgagg 1200
   cacctatctc agcgatctgt ctatttcgtt catccatagt tgcctgactc cccgtcgtgt 1260
   agataactac gatacgggag ggcttaccat ctggccccag tgctgcaatg ataccgcgag 1320
   acccacgctc accggctcca gatttatcag caataaacca gccagccgga agggccgagc 1380
   gcagaagtgg tcctgcaact ttatccgcct ccatccagtc tattaattgt tgccgggaag 1440
   ctagagtaag tagttcgcca gttaatagtt tgcgcaacgt tgttgccatt gctacaggca 1500
   tcgtggtgtc acgctcgtcg tttggtatgg cttcattcag ctccggttcc caacgatcaa 1560
   ggcgagttac atgatccccc atgttgtgca aaaaagcggt tagctccttc ggtcctccga 1620
   tcgttgtcag aagtaagttg gccgcagtgt tatcactcat ggttatggca gcactgcata 1680
   attctcttac tgtcatgcca tccgtaagat gcttttctgt gactggtgag tactcaacca 1740
   agtcattctg agaatagtgt atgcggcgac cgagttgctc ttgcccggcg tcaatacggg 1800
   ataataccgc gccacatagc agaactttaa aagtgctcat cattggaaaa cgttcttcgg 1860
   ggcgaaaact ctcaaggatc ttaccgctgt tgagatccag ttcgatgtaa cccactcgtg 1920
   cacccaactg atcttcagca tcttttactt tcaccagcgt ttctgggtga gcaaaaacag 1980
   gaaggcaaaa tgccgcaaaa aagggaataa gggcgacacg gaaatgttga atactcatac 2040
   tcttcctttt tcaatattat tgaagcattt atcagggtta ttgtctcatg agcggataca      2100
   tatttgaatg tatttagaaa aataaacaaa taggggttcc gcgcacattt ccccgaaaag      2160
   tgccacctga cgtctaagaa accattatta tcatgacatt aacctataaa aataggcgta      2220
   tcacgaggcc ctttcgtctc gcgcgtttcg gtgatgacgg tgaaaacctc tgacacatgc      2280
   agctcccgga gacggtcaca gcttgtctgt aagcggatgc cgggagcaga caagcccgtc      2340
   agggcgcgtc agcgggtgtt ggcgggtgtc ggggctggct taactatgcg gcatcagagc      2400
   agattgtact gagagtgcac catatgggta ccgagctcgc ggccgcaagc cagctggagc      2460
   tgttgacaat taatcatcgg ctcgtataat gtgtgggaat tcgagctcgg atcctctaga      2520
   ctcgagggaa gcttccacac attatacgag ccgatgatta attgtcaaca gctcgatatc      2580
   atcagctg                                                               2588
<210> 17
   <211> 316
   <212> DNA
   <213> Frankliniella occidentalis
<400> 17
   tctagagcag atgccatcag acaagacaat tggaggaggt gatgattctt ttaacacatt        60
   cttcagcgag acgggcgctg gcaaacacgt ccccagggct gtctttgttg acctggaacc       120
   cactgtagtt gatgaggtcc gcactggtac ctaccgccag ctcttccacc ctgagcagct       180
   tatcaccggc aaggaggatg ctgctaacaa ctatgctcgt ggtcactaca ccattggcaa       240
   ggaaattgtc gaccttgtat tggaccgcat ccgcaagttg gctgaccagt gcacaggtct       300
   tcaaggcttc tctaga                                                       316
<210> 18
   <211> 400
   <212> DNA
   <213> Frankliniella occidentalis
<400> 18
   tctagaggtt ctcgacaagc tgaaggctga gcgtgaacgt ggtatcacca ttgacattgc        60
   tctctggaag ttcgaaactc caaagtacaa cgtcactgtc atcgatgctc caggtcacag       120
   agatttcatt aagaacatga ttactggtac ctcccaagct gactgcgcta tcctcatcat       180
   tggtggtggt gttggtgaat tcgaagctgg tatctccaag gatggtcaaa ccagagaaca       240
   cgctcttctc tctttcaccc tcggtgtcag acaactcatt gtcgccgtca acaagatgga       300
   caccactcaa tactctgaag cccgtttcac tgaaattatc aaggaaaccc aaaacttcat       360
   caaggagggt cggttacaac cctaagacac cgcctctaga                             400
<210> 19
   <211> 2898
   <212> DNA
   <213> Artificial Sequence
<220>
   <223> pTub3
<400> 19
   acctgcggcc gcaagcttgg atccgaattc ctgtgtgaaa ttgttatccg ctcacaattc 60
   cacacaacat acgagccgga agcataaagt gtaaagcctg gggtgcctaa tgagtgagct 120
   aactcacatt aattgcgttg cgctcactgc ccgctttcca gtcgggaaac ctgtcgtgcc 180
   agctgcatta atgaatcggc caacgcgcgg ggagaggcgg tttgcgtatt gggcgctctt 240
   ccgcttcctc gctcactgac tcgctgcgct cggtcgttcg gctgcggcga gcggtatcag 300
   ctcactcaaa ggcggtaata cggttatcca cagaatcagg ggataacgca ggaaagaaca 360
   tgtgagcaaa aggccagcaa aaggccagga accgtaaaaa ggccgcgttg ctggcgtttt 420
   tccataggct ccgcccccct gacgagcatc acaaaaatcg acgctcaagt cagaggtggc 480
   gaaacccgac aggactataa agataccagg cgtttccccc tggaagctcc ctcgtgcgct 540
   ctcctgttcc gaccctgccg cttaccggat acctgtccgc ctttctccct tcgggaagcg 600
   tggcgctttc tcatagctca cgctgtaggt atctcagttc ggtgtaggtc gttcgctcca 660
   agctgggctg tgtgcacgaa ccccccgttc agcccgaccg ctgcgcctta tccggtaact 720
   atcgtcttga gtccaacccg gtaagacacg acttatcgcc actggcagca gccactggta 780
   acaggattag cagagcgagg tatgtaggcg gtgctacaga gttcttgaag tggtggccta 840
   actacggcta cactagaagg acagtatttg gtatctgcgc tctgctgaag ccagttacct 900
   tcggaaaaag agttggtagc tcttgatccg gcaaacaaac caccgctggt agcggtggtt 960
   tttttgtttg caagcagcag attacgcgca gaaaaaaagg atctcaagaa gatcctttga 1020
   tcttttctac ggggtctgac gctcagtgga acgaaaactc acgttaaggg attttggtca 1080
   tgagattatc aaaaaggatc ttcacctaga tccttttaaa ttaaaaatga agttttaaat 1140
   caatctaaag tatatatgag taaacttggt ctgacagtta ccaatgctta atcagtgagg 1200
   cacctatctc agcgatctgt ctatttcgtt catccatagt tgcctgactc cccgtcgtgt 1260
   agataactac gatacgggag ggcttaccat ctggccccag tgctgcaatg ataccgcgag 1320
   acccacgctc accggctcca gatttatcag caataaacca gccagccgga agggccgagc 1380
   gcagaagtgg tcctgcaact ttatccgcct ccatccagtc tattaattgt tgccgggaag 1440
   ctagagtaag tagttcgcca gttaatagtt tgcgcaacgt tgttgccatt gctacaggca 1500
   tcgtggtgtc acgctcgtcg tttggtatgg cttcattcag ctccggttcc caacgatcaa 1560
   ggcgagttac atgatccccc atgttgtgca aaaaagcggt tagctccttc ggtcctccga 1620
   tcgttgtcag aagtaagttg gccgcagtgt tatcactcat ggttatggca gcactgcata 1680
   attctcttac tgtcatgcca tccgtaagat gcttttctgt gactggtgag tactcaacca     1740
   agtcattctg agaatagtgt atgcggcgac cgagttgctc ttgcccggcg tcaatacggg     1800
   ataataccgc gccacatagc agaactttaa aagtgctcat cattggaaaa cgttcttcgg     1860
   ggcgaaaact ctcaaggatc ttaccgctgt tgagatccag ttcgatgtaa cccactcgtg     1920
   cacccaactg atcttcagca tcttttactt tcaccagcgt ttctgggtga gcaaaaacag     1980
   gaaggcaaaa tgccgcaaaa aagggaataa gggcgacacg gaaatgttga atactcatac     2040
   tcttcctttt tcaatattat tgaagcattt atcagggtta ttgtctcatg agcggataca     2100
   tatttgaatg tatttagaaa aataaacaaa taggggttcc gcgcacattt ccccgaaaag     2160
   tgccacctga cgtctaagaa accattatta tcatgacatt aacctataaa aataggcgta     2220
   tcacgaggcc ctttcgtctc gcgcgtttcg gtgatgacgg tgaaaacctc tgacacatgc     2280
   agctcccgga gacggtcaca gcttgtctgt aagcggatgc cgggagcaga caagcccgtc     2340
   agggcgcgtc agcgggtgtt ggcgggtgtc ggggctggct taactatgcg gcatcagagc     2400
   agattgtact gagagtgcac catatgggta ccgagctcgc ggccgcaagc cagctggagc     2460
   tgttgacaat taatcatcgg ctcgtataat gtgtgggaat tcgagctcgg atcctctaga     2520
   gcagatgcca tcagacaaga caattggagg aggtgatgat tcttttaaca cattcttcag     2580
   cgagacgggc gctggcaaac acgtccccag ggctgtcttt gttgacctgg aacccactgt     2640
   agttgatgag gtccgcactg gtacctaccg ccagctcttc caccctgagc agcttatcac     2700
   cggcaaggag gatgctgcta acaactatgc tcgtggtcac tacaccattg gcaaggaaat     2760
   tgtcgacctt gtattggacc gcatccgcaa gttggctgac cagtgcacag gtcttcaagg     2820
   cttctctaga ctcgagggaa gcttccacac attatacgag ccgatgatta attgtcaaca     2880
   gctcgatatc atcagctg                                                   2898
<210> 20
   <211> 2982
   <212> DNA
   <213> Artificial Sequence
<220>
   <223> pElong1
<400> 20
   acctgcggcc gcaagcttgg atccgaattc ctgtgtgaaa ttgttatccg ctcacaattc       60
   cacacaacat acgagccgga agcataaagt gtaaagcctg gggtgcctaa tgagtgagct      120
   aactcacatt aattgcgttg cgctcactgc ccgctttcca gtcgggaaac ctgtcgtgcc      180
   agctgcatta atgaatcggc caacgcgcgg ggagaggcgg tttgcgtatt gggcgctctt      240
   ccgcttcctc gctcactgac tcgctgcgct cggtcgttcg gctgcggcga gcggtatcag      300
   ctcactcaaa ggcggtaata cggttatcca cagaatcagg ggataacgca ggaaagaaca 360
   tgtgagcaaa aggccagcaa aaggccagga accgtaaaaa ggccgcgttg ctggcgtttt 420
   tccataggct ccgcccccct gacgagcatc acaaaaatcg acgctcaagt cagaggtggc 480
   gaaacccgac aggactataa agataccagg cgtttccccc tggaagctcc ctcgtgcgct 540
   ctcctgttcc gaccctgccg cttaccggat acctgtccgc ctttctccct tcgggaagcg 600
   tggcgctttc tcatagctca cgctgtaggt atctcagttc ggtgtaggtc gttcgctcca 660
   agctgggctg tgtgcacgaa ccccccgttc agcccgaccg ctgcgcctta tccggtaact 720
   atcgtcttga gtccaacccg gtaagacacg acttatcgcc actggcagca gccactggta 780
   acaggattag cagagcgagg tatgtaggcg gtgctacaga gttcttgaag tggtggccta 840
   actacggcta cactagaagg acagtatttg gtatctgcgc tctgctgaag ccagttacct 900
   tcggaaaaag agttggtagc tcttgatccg gcaaacaaac caccgctggt agcggtggtt 960
   tttttgtttg caagcagcag attacgcgca gaaaaaaagg atctcaagaa gatcctttga 1020
   tcttttctac ggggtctgac gctcagtgga acgaaaactc acgttaaggg attttggtca 1080
   tgagattatc aaaaaggatc ttcacctaga tccttttaaa ttaaaaatga agttttaaat 1140
   caatctaaag tatatatgag taaacttggt ctgacagtta ccaatgctta atcagtgagg 1200
   cacctatctc agcgatctgt ctatttcgtt catccatagt tgcctgactc cccgtcgtgt 1260
   agataactac gatacgggag ggcttaccat ctggccccag tgctgcaatg ataccgcgag 1320
   acccacgctc accggctcca gatttatcag caataaacca gccagccgga agggccgagc 1380
   gcagaagtgg tcctgcaact ttatccgcct ccatccagtc tattaattgt tgccgggaag 1440
   ctagagtaag tagttcgcca gttaatagtt tgcgcaacgt tgttgccatt gctacaggca 1500
   tcgtggtgtc acgctcgtcg tttggtatgg cttcattcag ctccggttcc caacgatcaa 1560
   ggcgagttac atgatccccc atgttgtgca aaaaagcggt tagctccttc ggtcctccga 1620
   tcgttgtcag aagtaagttg gccgcagtgt tatcactcat ggttatggca gcactgcata 1680
   attctcttac tgtcatgcca tccgtaagat gcttttctgt gactggtgag tactcaacca 1740
   agtcattctg agaatagtgt atgcggcgac cgagttgctc ttgcccggcg tcaatacggg 1800
   ataataccgc gccacatagc agaactttaa aagtgctcat cattggaaaa cgttcttcgg 1860
   ggcgaaaact ctcaaggatc ttaccgctgt tgagatccag ttcgatgtaa cccactcgtg 1920
   cacccaactg atcttcagca tcttttactt tcaccagcgt ttctgggtga gcaaaaacag 1980
   gaaggcaaaa tgccgcaaaa aagggaataa gggcgacacg gaaatgttga atactcatac 2040
   tcttcctttt tcaatattat tgaagcattt atcagggtta ttgtctcatg agcggataca 2100
   tatttgaatg tatttagaaa aataaacaaa taggggttcc gcgcacattt ccccgaaaag 2160
   tgccacctga cgtctaagaa accattatta tcatgacatt aacctataaa aataggcgta 2220
   tcacgaggcc ctttcgtctc gcgcgtttcg gtgatgacgg tgaaaacctc tgacacatgc 2280
   agctcccgga gacggtcaca gcttgtctgt aagcggatgc cgggagcaga caagcccgtc 2340
   agggcgcgtc agcgggtgtt ggcgggtgtc ggggctggct taactatgcg gcatcagagc 2400
   agattgtact gagagtgcac catatgggta ccgagctcgc ggccgcaagc cagctggagc 2460
   tgttgacaat taatcatcgg ctcgtataat gtgtgggaat tcgagctcgg atcctctaga 2520
   ggttctcgac aagctgaagg ctgagcgtga acgtggtatc accattgaca ttgctctctg 2580
   gaagttcgaa actccaaagt acaacgtcac tgtcatcgat gctccaggtc acagagattt 2640
   cattaagaac atgattactg gtacctccca agctgactgc gctatcctca tcattggtgg 2700
   tggtgttggt gaattcgaag ctggtatctc caaggatggt caaaccagag aacacgctct 2760
   tctctctttc accctcggtg tcagacaact cattgtcgcc gtcaacaaga tggacaccac 2820
   tcaatactct gaagcccgtt tcactgaaat tatcaaggaa acccaaaact tcatcaagga 2880
   gggtcggtta caaccctaag acaccgcctc tagactcgag ggaagcttcc acacattata 2940
   cgagccgatg attaattgtc aacagctcga tatcatcagc tg 2982
<210> 21
   <211> 722
   <212> DNA
   <213> Artificial Sequence
<220>
   <223> RNAseIII Bfo2
<400> 21
   gttgatacag ccctgtttca tgtgcaatta acagacgcaa agaataactg gaacagcatg      60
   aaccccattc ttataaataa gctgcagcgt aaactaggct acacttttac tcatccggaa     120
   cttctgcaac aagccttgac gcatcgcagt gccagtagta aacataatga aagattagaa     180
   tttcttgggg attcaatctt aagctatgtc atcgcgaatg cgctttattg tcaattccct     240
   cgtgttgatg aaggggatat gagccggatg cgtgcgacct tggttagggg gaataccctt     300
   gctgagatgg cacgggagtt cgaactgggt gagtgcttac gcctaggccc tggtgagctt     360
   aagagtggtg gatttcgccg tgaatccatt ttagcggata ccgtagaagc actgattggc     420
   ggaattttct tggatagcga tatccaacaa accgaaaaat taatccttaa ttggtatcag     480
   tcgcggctgg aagcgatcag cccgggtgat aagcaaaaag atccgaaaac acgtttacaa     540
   gagtatcttc aagggcgtca cttaccgtta ccggcctatt tagtggttca ggtgagggga     600
   gaggctcatg atcaagaatt tacgattcac tgccaagtga gtggtatgaa tgaagcggtg     660
   gttggcattg gttctagccg tcgtaaagca gaacaggctg ctgcagagca agcactgact     720
   aa                                                                    722
<210> 22
   <211> 3384
   <212> DNA
   <213> Artificial Sequence
<220>
   <223> pRNA1
<400> 22
   gacgaaaggg cctcgtgata cgcctatttt tataggttaa tgtcatgata ataatggttt      60
   cttagacgtc aggtggcact tttcggggaa atgtgcgcgg aacccctatt tgtttatttt     120
   tctaaataca ttcaaatatg tatccgctca tgagacaata accctgataa atgcttcaat     180
   aatattgaaa aaggaagagt atgagtattc aacatttccg tgtcgccctt attccctttt     240
   ttgcggcatt ttgccttcct gtttttgctc acccagaaac gctggtgaaa gtaaaagatg     300
   ctgaagatca gttgggtgca cgagtgggtt acatcgaact ggatctcaac agcggtaaga     360
   tccttgagag ttttcgcccc gaagaacgtt ttccaatgat gagcactttt aaagttctgc     420
   tatgtggcgc ggtattatcc cgtattgacg ccgggcaaga gcaactcggt cgccgcatac     480
   actattctca gaatgacttg gttgagtact caccagtcac agaaaagcat cttacggatg     540
   gcatgacagt aagagaatta tgcagtgctg ccataaccat gagtgataac actgcggcca     600
   acttacttct gacaacgatc ggaggaccga aggagctaac cgcttttttg cacaacatgg     660
   gggatcatgt aactcgcctt gatcgttggg aaccggagct gaatgaagcc ataccaaacg     720
   acgagcgtga caccacgatg cctgtagcaa tggcaacaac gttgcgcaaa ctattaactg     780
   gcgaactact tactctagct tcccggcaac aattaataga ctggatggag gcggataaag     840
   ttgcaggacc acttctgcgc tcggcccttc cggctggctg gtttattgct gataaatctg     900
   gagccggtga gcgtgggtct cgcggtatca ttgcagcact ggggccagat ggtaagccct     960
   cccgtatcgt agttatctac acgacgggga gtcaggcaac tatggatgaa cgaaatagac    1020
   agatcgctga gataggtgcc tcactgatta agcattggta actgtcagac caagtttact    1080
   catatatact ttagattgat ttaaaacttc atttttaatt taaaaggatc taggtgaaga    1140
   tcctttttga taatctcatg accaaaatcc cttaacgtga gttttcgttc cactgagcgt    1200
   cagaccccgt agaaaagatc aaaggatctt cttgagatcc tttttttctg cgcgtaatct    1260
   gctgcttgca aacaaaaaaa ccaccgctac cagcggtggt ttgtttgccg gatcaagagc    1320
   taccaactct ttttccgaag gtaactggct tcagcagagc gcagatacca aatactgtcc    1380
   ttctagtgta gccgtagtta ggccaccact tcaagaactc tgtagcaccg cctacatacc    1440
   tcgctctgct aatcctgtta ccagtggctg ctgccagtgg cgataagtcg tgtcttaccg    1500
   ggttggactc aagacgatag ttaccggata aggcgcagcg gtcgggctga acggggggtt    1560
   cgtgcacaca gcccagcttg gagcgaacga cctacaccga actgagatac ctacagcgtg    1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cagatctggg gaattcgttg atacagccct gtttcatgtg caattaacag 2280
   acgcaaagaa taactggaac agcatgaacc ccattcttat aaataagctg cagcgtaaac 2340
   taggctacac ttttactcat ccggaacttc tgcaacaagc cttgacgcat cgcagtgcca 2400
   gtagtaaaca taatgaaaga ttagaatttc ttggggattc aatcttaagc tatgtcatcg 2460
   cgaatgcgct ttattgtcaa ttccctcgtg ttgatgaagg ggatatgagc cggatgcgtg 2520
   cgaccttggt tagggggaat acccttgctg agatggcacg ggagttcgaa ctgggtgagt 2580
   gcttacgcct aggccctggt gagcttaaga gtggtggatt tcgccgtgaa tccattttag 2640
   cggataccgt agaagcactg attggcggaa ttttcttgga tagcgatatc caacaaaccg 2700
   aaaaattaat ccttaattgg tatcagtcgc ggctggaagc gatcagcccg ggtgataagc 2760
   aaaaagatcc gaaaacacgt ttacaagagt atcttcaagg gcgtcactta ccgttaccgg 2820
   cctatttagt ggttcaggtg aggggagagg ctcatgatca agaatttacg attcactgcc 2880
   aagtgagtgg tatgaatgaa gcggtggttg gcattggttc tagccgtcgt aaagcagaac 2940
   aggctgctgc agagcaagca ctgactaaaa gcttggctgc agatctggca ctggccgtcg 3000
   ttttacaacg tcgtgactgg gaaaaccctg gcgttaccca acttaatcgc cttgcagcac 3060
   atcccccttt cgccagctgg cgtaatagcg aagaggcccg caccgatcgc ccttcccaac 3120
   agttgcgcag cctgaatggc gaatggcgcc tgatgcggta ttttctcctt acgcatctgt 3180
   gcggtatttc acaccgcata tggtgcactc tcagtacaat ctgctctgat gccgcatagt 3240
   taagccagcc ccgacacccg ccaacacccg ctgacgcgcc ctgacgggct tgtctgctcc 3300
   cggcatccgc ttacagacaa gctgtgaccg tctccgggag ctgcatgtgt cagaggtttt 3360
   caccgtcatc accgaaacgc gcga 3384
<210> 23
   <211> 5109
   <212> DNA
   <213> Artificial Sequence
<220>
   <223> pRNA2
<400> 23
   gacgaaaggg cctcgtgata cgcctatttt tataggttaa tgtcatgata ataatggttt        60
   cttagacgtc aggtggcact tttcggggaa atgtgcgcgg aacccctatt tgtttatttt       120
   tctaaataca ttcaaatatg tatccgctca tgagacaata accctgataa atgcttcaat       180
   aatattgaaa aaggaagagt atgagtattc aacatttccg tgtcgccctt attccctttt       240
   ttgcggcatt ttgccttcct gtttttgctc acccagaaac gctggtgaaa gtaaaagatg       300
   ctgaagatca gttgggtgca cgagtgggtt acatcgaact ggatctcaac agcggtaaga       360
   tccttgagag ttttcgcccc gaagaacgtt ttccaatgat gagcactttt aaagttctgc       420
   tatgtggcgc ggtattatcc cgtattgacg ccgggcaaga gcaactcggt cgccgcatac       480
   actattctca gaatgacttg gttgagtact caccagtcac agaaaagcat cttacggatg       540
   gcatgacagt aagagaatta tgcagtgctg ccataaccat gagtgataac actgcggcca       600
   acttacttct gacaacgatc ggaggaccga aggagctaac cgcttttttg cacaacatgg       660
   gggatcatgt aactcgcctt gatcgttggg aaccggagct gaatgaagcc ataccaaacg       720
   acgagcgtga caccacgatg cctgtagcaa tggcaacaac gttgcgcaaa ctattaactg       780
   gcgaactact tactctagct tcccggcaac aattaataga ctggatggag gcggataaag       840
   ttgcaggacc acttctgcgc tcggcccttc cggctggctg gtttattgct gataaatctg       900
   gagccggtga gcgtgggtct cgcggtatca ttgcagcact ggggccagat ggtaagccct       960
   cccgtatcgt agttatctac acgacgggga gtcaggcaac tatggatgaa cgaaatagac      1020
   agatcgctga gataggtgcc tcactgatta agcattggta actgtcagac caagtttact      1080
   catatatact ttagattgat ttaaaacttc atttttaatt taaaaggatc taggtgaaga      1140
   tcctttttga taatctcatg accaaaatcc cttaacgtga gttttcgttc cactgagcgt      1200
   cagaccccgt agaaaagatc aaaggatctt cttgagatcc tttttttctg cgcgtaatct      1260
   gctgcttgca aacaaaaaaa ccaccgctac cagcggtggt ttgtttgccg gatcaagagc      1320
   taccaactct ttttccgaag gtaactggct tcagcagagc gcagatacca aatactgtcc      1380
   ttctagtgta gccgtagtta ggccaccact tcaagaactc tgtagcaccg cctacatacc      1440
   tcgctctgct aatcctgtta ccagtggctg ctgccagtgg cgataagtcg tgtcttaccg      1500
   ggttggactc aagacgatag ttaccggata aggcgcagcg gtcgggctga acggggggtt      1560
   cgtgcacaca gcccagcttg gagcgaacga cctacaccga actgagatac ctacagcgtg      1620
   agctatgaga aagcgccacg cttcccgaag ggagaaaggc ggacaggtat ccggtaagcg      1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cagatctggg gaattcgttg atacagccct gtttcatgtg caattaacag 2280
   acgcaaagaa taactggaac agcatgaacc ccattcttat aaataagctg cagcgtaaac 2340
   taggctacac ttttactcat ccggaacttc tgcaacaagc cttgacgcat cgcagtgcca 2400
   gtagtaaaca taatgaaaga ttagaatttc ttggggattc aatcttaagc tatgtcatcg 2460
   cgaatgcgct ttattgtcaa ttccctcgtg ttgatgaagg ggatatgagc cggatgcgtg 2520
   cgaccttggt tagggggaat acccttgctg agatggcacg ggagttcgaa ctgggtgagt 2580
   gcttacgcct aggccctggt gagcttaaga gtggtggatt tcgccgtgaa tccattttag 2640
   cggataccgt agaagcactg attggcggaa ttttcttgga tagcgatagc tttatgcttg 2700
   taaaccgttt tgtgaaaaaa tttttaaaat aaaaaagggg acctctaggg tccccaatta 2760
   attagtaata taatctatta aaggtcattc aaaaggtcat ccaccggatc aattcccctg 2820
   ctcgcgcagg ctgggtgcca agctctcggg taacatcaag gcccgatcct tggagccctt 2880
   gccctcccgc acgatgatcg tgccgtgatc gaaatccaga tccttgaccc gcagttgcaa 2940
   accctcactg atccggctca cggtaactga tgccgtattt gcagtaccag cgtacggccc 3000
   acagaatgat gtcacgctga aaatgccggc ctttgaatgg gttcatgtgc agctccatca 3060
   gcaaaagggg atgataagtt tatcaccacc gactatttgc aacagtgccg ttgatcgtgc 3120
   tatgatcgac tgatgtcatc agcggtggag tgcaatgtcg tgcaatacga atggcgaaaa 3180
   gccgagctca tcggtcagct tctcaacctt ggggttaccc ccggcggtgt gctgctggtc 3240
   cacagctcct tccgtagcgt ccggcccctc gaagatgggc cacttggact gatcgaggcc 3300
   ctgcgtgctg cgctgggtcc gggagggacg ctcgtcatgc cctcgtggtc aggtctggac 3360
   gacgagccgt tcgatcctgc cacgtcgccc gttacaccgg accttggagt tgtctctgac 3420
   acattctggc gcctgccaaa tgtaaagcgc agcgcccatc catttgcctt tgcggcagcg 3480
   gggccacagg cagagcagat catctctgat ccattgcccc tgccacctca ctcgcctgca 3540
   agcccggtcg cccgtgtcca tgaactcgat gggcaggtac ttctcctcgg cgtgggacac 3600
   gatgccaaca cgacgctgca tcttgccgag ttgatggcaa aggttcccta tggggtgccg 3660
   agacactgca ccattcttca ggatggcaag ttggtacgcg tcgattatct cgagaatgac 3720
   cactgctgtg agcgctttgc cttggcggac aggtggctca aggagaagag ccttcagaag 3780
   gaaggtccag tcggtcatgc ctttgctcgg ttgatccgct cccgcgacat tgtggcgaca 3840
   gccctgggtc aactgggccg agatccgttg atcttcctgc atccgccaga gggcgggatg 3900
   cgaagaatgc gatgccgctc gccagtcgat tggctgagct catgagcgga gaacgagatg 3960
   acgttggagg ggcaaggtcg cgctgattgc tggggcaaca cgtgaaaggc gagatcacca 4020
   aggtagtcgg caaataatgt ctaacaattc gttcaagccg acgccgcttc gcggcgcggc 4080
   ttaactcaag cgttagatgc actaagcaca taattgctca cagccaaact atcaggtcaa 4140
   gtctgctttt attattttta agcgtgcata ataagcccta cacaaattgg gagatatatc 4200
   atgaaaggct ggctttttct tgttatcgca atagttggcg aagtaatcgc aacatccgca 4260
   ttaaaatcta gcgagggctt tactaagctg atccggtgga tgaccttttg aatgaccttt 4320
   aatagattat attactaatt aattggggac cctagaggtc ccctttttta ttttaaaaat 4380
   tttttcacaa aacggtttac aagcataaag ctatccaaca aaccgaaaaa ttaatcctta 4440
   attggtatca gtcgcggctg gaagcgatca gcccgggtga taagcaaaaa gatccgaaaa 4500
   cacgtttaca agagtatctt caagggcgtc acttaccgtt accggcctat ttagtggttc 4560
   aggtgagggg agaggctcat gatcaagaat ttacgattca ctgccaagtg agtggtatga 4620
   atgaagcggt ggttggcatt ggttctagcc gtcgtaaagc agaacaggct gctgcagagc 4680
   aagcactgac taaaagcttg gctgcagatc tggcactggc cgtcgtttta caacgtcgtg 4740
   actgggaaaa ccctggcgtt acccaactta atcgccttgc agcacatccc cctttcgcca 4800
   gctggcgtaa tagcgaagag gcccgcaccg atcgcccttc ccaacagttg cgcagcctga 4860
   atggcgaatg gcgcctgatg cggtattttc tccttacgca tctgtgcggt atttcacacc 4920
   gcatatggtg cactctcagt acaatctgct ctgatgccgc atagttaagc cagccccgac 4980
   acccgccaac acccgctgac gcgccctgac gggcttgtct gctcccggca tccgcttaca 5040
   gacaagctgt gaccgtctcc gggagctgca tgtgtcagag gttttcaccg tcatcaccga 5100
   aacgcgcga 5109